# EUROPEAN PATENT APPLICATION

(11) **EP 1 783 204 A2**
(43) Date of publication of application: **09.05.2007**
(21) Application number: 06026212.8
(22) Date of filing: 18.12.2006
(51) Int. Cl.: C12N 5/00, G01N 21/23

(54) **Biological sample handling and imaging**

(30) Priority: 04.04.2006 US 397336
(71) Applicant: Cambridge Research & Instrumentation, Inc., Woburn, MA 01801 (US)
(72) Inventor: Hoyt, Clifford C., Wellesley MA 02481 (US); Boutin, Cathy, New Ipswich NH 03071 (US)
(74) Representative: Epping - Hermann - Fischer

(57) **Abstract**

Disclosed are methods and systems for assessing the developmental potential of an oocyte based on one or more quantitative metrics derived from one or more birefringence images of a sample comprising the oocyte, wherein the assessing comprises comparing the one or more quantitative metrics to reference information.

## Description

### BACKGROUND

A biological sample can be imaged under magnification to investigate structural elements within the sample. Images obtained under magnification can be recorded while the sample is mounted in a microscope. A variety of different imaging techniques can be used to obtain the images. For example, broad- or narrow-bandwidth light sources can be used to illuminate the sample, and light transmitted through the sample can be viewed by an operator through a microscope eyepiece and/or can be directed to a detector such as a CCD camera. In some samples, certain structural elements within the sample can be difficult to image with particular techniques because the contrast between the elements of interest and the rest of the sample may be small.

Birefringence imaging techniques can be used to provide contrast between structural elements of interest and the rest of the sample. For example, birefringence imaging techniques may provide contrast even when the elements appear approximately transparent in images recorded using other techniques, *e.g.*, broadband illumination. A sample is "birefringent" when it has an anisotropic index of refraction. Such samples can be oriented with respect to a propagation direction of light through the sample so that light having a first polarization direction (transverse to the propagation direction) will experience an *ordinary* refractive index *nₒ* on passing through the sample, and light having a second polarization direction orthogonal to the first polarization direction (and also transverse to the propagation direction) will experience an *extraordinary* refractive index *nₑ* that is different from *nₒ.* Light having a polarization direction that is intermediate between the first and second polarization directions will have the component along the first polarization direction experience the ordinary refractive index and the component along the second polarization direction experience the extraordinary refractive index. Upon passing through the sample, one polarization component will have a phase delay (*i.e.,* be "retarded") relative to the other polarization component, and such delay can change the state of polarization of the light.

In polycrystalline and amorphous samples, such as biological medium, different volume elements of the sample along the light propagation direction can have different birefringence properties. For example, the orientation of the axes for the ordinary and extraordinary indices of refraction might vary along the light path. The overall effect, however, can generally be expressed by an equivalent set of birefringence properties having constant indices and orientation axes for the ordinary and extraordinary refraction. The "retardance" of the sample for such incident light passing through the sample is the integrated phase delay along the light path, typically expressed in waves or nanometers, between light components polarized along the axes for the ordinary and extraordinary indices of refraction axes corresponding to this equivalent set of birefringence properties As used herein, therefore, retardance is an extensive property that depends on the relative orientation of the sample with respect to the incident light and the thickness of the sample through which the light passes.

In birefringent imaging systems, input light is incident on the sample with a selected polarization, which is then altered by the presence of birefringence in the sample. Output polarization analyzers can then be used to create contrast (*e.g.*, by polarization-dependent attenuation) in an image of the sample. For example, different regions of the sample through which corresponding portions of the input light pass exhibit different amounts of retardation, and thereby changes to the state of polarization of the different input light portions being imaged. Birefringence imaging systems are disclosed, for example, in U.S. Patent No. 5,521,705 entitled "POLARIZED LIGHT MICROSCOPY" by Rudolf Oldenbourg and Guang Mei, filed on May 12, 1994, the entire contents of which are incorporated herein by reference. Birefringence imaging systems are further disclosed, for example, in U.S. Patent No. 6,924,893 entitled "ENHANCING POLARIZED LIGHT MICROSCOPY" by Rudolf Oldenbourg *et al.,* filed on May 12, 2003, the entire contents of which are incorporated herein by reference.

As an example, images of mammalian oocytes can be obtained using birefringence imaging techniques. Oocytes are formed during fetal life in mammals and may remain suspended at the diplotene stage of meiosis for many years before beginning to grow, resuming meiosis, and undergoing fertilization. This extended developmental history can sometimes produce defective mature oocytes that lack the capacity to engender the development of healthy zygotes. Therefore, the odds of successful outcomes for therapies employing assisted reproductive technology (ART) methods may be increased by identifying and pre-selecting non-defective oocytes.

### SUMMARY

In one aspect, the invention features a method that includes assessing the developmental potential of an oocyte based on one or more quantitative metrics derived from one or more birefringence images of a sample that includes the oocyte, where the assessing includes comparing the one or more quantitative metrics to reference information.

Embodiments of the method can include any of the following features.

The reference information can be derived from one or more birefringence images of a reference sample, where the birefringence images of the first mentioned sample and the reference sample are obtained under like conditions. The reference information can include one or more reference values. The one or more reference values can include one or more predetermined thresholds. The one or more predetermined thresholds can be provided by a user prior to obtaining the birefringence image, for example.

Alternatively, or in addition, the reference information can include one or more reference images. For example, the reference information can be a series of reference images that are birefringent images indicative of oocytes with different developmental potentials. In such embodiments, for example, a user and/or an electronic processor by way of automated machine vision techniques can compare the birefringence image of a present sample to those in the, reference series to determine the best match and thereby assign a grade corresponding to the that of the matching reference image. Furthermore, in other embodiments, reference values, including threshold values, can be displayed on a reference image using black-white, false-color, or other methods used to visually display information.

The method can further include assessing the developmental potential of an additional oocyte sample based on one or more quantitative metrics derived from one or more birefringence images of the additional oocyte, where the assessing includes comparing the one or more quantitative metrics for the additional oocyte to the same reference information as that for the assessment of the first mentioned oocyte sample.

The oocyte can be a primary oocyte for example, or a secondary oocyte. The oocyte can be a fertilized oocyte. The oocyte can be a mammalian oocyte, *e.g.,* a human oocyte. Alternatively, the mammal can be an ape, a monkey, a bovine, a horse, a pig, a sheep, a canine, a feline, or a rodent.

The one or more quantitative metrics can be derived from a birefringence image of at least one structural entity in the oocyte. The structural entity can be an organelle of the oocyte. For example, the organelle can be a meiotic spindle. As another example, the organelle can be a zona pellucida. The structural entity can be at least one of: a meiotic spindle, a zona pellucida, a cytoplasm, an oolemma, and a polar body.

Assessing the developmental potential of the oocyte can include grading the oocyte into one of three or more tiers based on the one or more quantitative metrics.

The assessment can be made automatically by an electronic processor. Alternatively, or in combination with at least a partial automated assessment, a user can make the assessment. Further, the assessment can be made based on a combination of quantitative metrics. The quantitative metrics can be combined according to a mathematical algorithm.

The one or more quantitative metrics can include any of a spatial location and a size of a structural entity of the oocyte in the birefringence image.

The one or more quantitative metrics can include dimensional information about a meiotic spindle of the oocyte. For example, the one or more quantitative metrics can include a length along a polar axis of a meiotic spindle of the oocyte, and/or a width perpendicular to a polar axis of a meiotic spindle of the oocyte, and/or a cross-sectional area of a meiotic spindle of the oocyte, and/or a ratio of a length along a polar axis of a meiotic spindle of the oocyte to a nearest point in a zona pellucida of the oocyte, and/or an angle between a polar axis of a meiotic spindle of the oocyte and a line extending from a center of the oocyte to a zona pellucida of the oocyte, and/or a total retardance of a meiotic spindle of an oocyte, and/or a distribution of spatial frequencies corresponding to a spatial variation of retardance of a meiotic spindle of an oocyte.

The one or more quantitative metrics can include dimensional information about a zona pellucida of the oocyte. For example, the one or more quantitative metrics can include a thickness of one or more layers of a zona pellucida of an oocyte, and/or a total retardance of one or more layers of a zona pellucida of an oocyte, and/or a maximum retardance of one or more layers of a zona pellucida of an oocyte.

The one or more quantitative metrics can include dimensional information about a cytoplasm of the oocyte. For example, the one or more quantitative metrics can include an average retardance of a cytoplasm of an oocyte, and/or a distribution of spatial frequencies corresponding to a spatial variation of retardance of a cytoplasm of an oocyte.

The one or more quantitative metrics can be determined automatically by an electronic processor.

The one or more birefringence images can be obtained using a birefringence imaging system comprising a microscope.

The one or more birefringence images can be obtained with a polar axis of a meiotic spindle of the oocyte substantially aligned in an object plane of a birefringence imaging system used to obtain the one or more birefringence images.

The one or more birefringence images can be obtained with a polar axis of a meiotic spindle of the oocyte forming an angle with respect to an object plane of a birefringence imaging system used to obtain the birefringence images, where the angle is less than about 30 degrees.

The one or more birefringence images can be obtained with an equatorial plane of the oocyte substantially aligned with an object plane of a birefringence imaging system. A cross-sectional area of the oocyte can be a local maximum in a birefringence image obtained when the equatorial plane of the oocyte and the object plane of the birefringence imaging system are substantially aligned. The oocyte can be substantially spherical.

In another aspect, the invention features an apparatus that includes: (a) a camera for use with a microscope birefringence imaging system to obtain a birefringence image of a sample that includes an oocyte; and (b) an electronic processor coupled to the camera and configured to store reference information for use in assessing the developmental potential of the oocyte based on one or more quantitative metrics derived from the birefringence image, where the assessing includes comparing the one or more quantitative metrics to the reference information.

Embodiments of the apparatus can include any of the following features.

The electronic processor can be configured to calculate the one or more quantitative metrics.

The electronic processor can be configured to perform the comparison of the one or more quantitative metrics to the reference information to make the assessment. The electronic processor can be configured to grade the oocyte into one of three or more tiers based on the one or more quantitative metrics.

The reference information can include one or more reference values. The one or more reference values can include one or more predetermined thresholds.

The reference values can be derived from one or more birefringence images of a reference sample, where the birefringence images of the first mentioned sample and the reference sample are obtained under like conditions.

The reference information can include one or more reference images.

The oocyte can be a secondary oocyte. Further, the oocyte can be a human oocyte.

The quantitative metrics can be derived from a birefringence image of at least one structural entity in the oocyte. For example, the structural entity can be a meiotic spindle. As another example, the structural entity can be a zona pellucida.

The one or more quantitative metrics can be derived from a birefringence image of a meiotic spindle of the oocyte, and/or from a birefringence image of a zona pellucida of the oocyte, and/or from a birefringence image of a cytoplasm of the oocyte, and/or from a birefringence image of an oolemma of the oocyte, and/or from a birefringence image of a polar body of the oocyte.

The one or more quantitative metrics can include dimensional information about a structural entity of the oocyte. The dimensional information can include at least one of a size, a length, an area, an orientation, a position, and a presence or absence of the structural entity.

The one or more quantitative metrics can include retardance information about a structural entity of the oocyte.

The birefringence image can be obtained with a polar axis of a meiotic spindle of the oocyte substantially aligned in an object plane of the imaging system.

The birefringence image can be obtained with a polar axis of a meiotic spindle of the oocyte forming an angle with respect to an object plane of the imaging system, where the angle is less than about 30 degrees.

The birefringence image can be obtained with an equatorial plane of the oocyte substantially aligned with an object plane of the imaging system.

The apparatus can further include the microscope birefringence imaging system.

In another aspect, the invention features a microscope imaging method that includes positioning a biological sample within a field of view of a microscope having an optical axis, and rotating the biological sample about a rotation axis different from the optical axis to improve an image of the sample produced by the microscope.

Embodiments of the method can include any of the following features.

The method can further include using a pipette to secure and position the sample within the field of view of the microscope. The pipette can be oriented at an angle with respect to an object plane of the microscope. The angle can be between about 5 degrees and about 30 degrees. The rotation axis can correspond to a longitudinal axis of the pipette.

Improving the image can include improving a resolution of a selected portion of the sample in the image. Alternatively, or in addition, improving the image can include improving a contrast of a selected portion of the sample in the image with respect to other portions of the sample in the image.

The image can be a birefringence image. Alternatively, or in addition, the image can be a fluorescence image.

The sample can include a cell.

The sample can include an oocyte. The sample can be rotated to improve an alignment between a polar axis of a meiotic spindle of the oocyte and an object plane of the microscope. For example, the sample can be rotated to substantially align the polar axis with the object plane. Alternatively, for example, the sample can be rotated so that an angle between the polar axis and the object plane is less than about 20 degrees.

The method can further include translating the sample to compensate for changes in a position of the sample relative to the microscope caused by the rotation. The translating can be performed on the basis of a calibration of the pipette. Further, the translating can be performed automatically.

In another aspect, the invention features an apparatus that includes a microscope configured to image a biological sample, where the microscope has an optical axis and includes a rotation element configured to position the sample within a field of view of a microscope and to adjustably rotate the biological sample about a rotation axis different from the optical axis to improve an image of the sample produced by the microscope.

Embodiments of the apparatus can include suitable features disclosed for any of the other apparatus.

In another aspect, the invention features an apparatus that includes a pipette that has an extended hollow portion and a mount portion mechanically coupled to the extended hollow portion, where the extended hollow portion is configured to secure a sample to one of its ends through the use of a vacuum applied to its other end, and the mount portion is configured to allow the extended hollow portion to rotate axially and to fix a position of the extended hollow portion with respect to one or more other degrees of freedom.

Embodiments of the apparatus can include any of the following features. The mount portion can surround at least a portion of the extended hollow portion.

An inner diameter of the extended hollow portion at the end used to secure the sample can be about 50 microns or less.

The extended hollow portion can include a reusable portion and a disposable portion secured to the reusable portion through a fitting in the mount portion. An outer diameter of the disposable portion can be about 100 microns or less. The reusable portion can be cylindrical.

The mount portion can include a plurality of seals configured to maintain a vacuum in the hollow extended portion.

The apparatus can include a motor coupled to the hollow extended portion and configured to axially rotate the hollow extended portion with respect to the mount portion.

The apparatus can include a translation stage coupled to the mount portion and configured to translate the sample end along one or more directions. The apparatus can also include an electronic processor coupled to the translation stage and configured to cause the translation stage to adjust the position of the sample end of the extended hollow portion to compensate for changes in its position caused by axial rotation of the extended hollow portion. The adjustments can be made based on precalibrated data. Alternatively, or in addition, the adjustments can be made based on analysis of microscope images of the pipette sample end. The analysis and adjustments can be performed as part of a feedback loop.

The apparatus can include a microscope system configured to produce images of the sample. The microscope system can produce birefringence images of the sample. The pipette can be tilted with respect to an object plane of the microscope system. The pipette can be configured to rotate the sample to improve a resolution of a selected portion of the sample in the images. The sample can include an oocyte, for example, and the selected portion can include a meiotic spindle of the oocyte.

Unless otherwise defined herein, all technical and scientific terms used herein have the same meaning as commonly understood by one of ordinary skill in the art to which this invention belongs.

The details of one or more embodiments of the invention are set forth in the accompanying drawings and the description below. Other features and advantages of the invention will be apparent from the description, drawings, and claims.

### DESCRIPTION OF DRAWINGS

**FIG. 1** is a schematic diagram of an embodiment of a birefringence imaging system.
**FIG. 2** is a perspective view of an embodiment of a sample manipulator.
**FIG. 3** is a cross-sectional view of the sample manipulator of **FIG. 2**.
**FIG. 4** is a schematic diagram showing positioning of a sample relative to a sample manipulator.
**FIG. 5** is a flow chart showing steps involved in calibrating a sample manipulator to correct for rotation-dependent translation of a sample.
**FIG. 6** is a schematic diagram of a mammalian oocyte.
**FIG. 7** is a flow chart showing steps involved in assessing developmental potential of a sample based on birefringence images.

Like reference symbols in the various drawings indicate like elements.

### DETAILED DESCRIPTION

Reproducibility and/or resolution in birefringence images can be improved for some samples by suitable positioning of the samples with respect to illumination light from an optical source. We disclose apparatus, systems, and methods for handling and orienting the samples in order to improve contrast in images obtained using birefringence imaging systems.

As disclosed herein, a "birefringence image" of a sample is one in which image colors, intensity values, or other pixel-to-pixel variations correspond, at least in part, to retardance at locations within the sample that correspond to the pixel positions. For example, a birefringence image can be a cross-sectional view of a sample measured in an object plane of an imaging system orthogonal to a propagation direction of illumination light. Each pixel value in the birefringence image can correspond to the retardance of the sample measured along the propagation direction, and in sample spatial regions that correspond to the pixel location. Birefringence images of the sample can be displayed as grayscale intensity maps, where the grayscale intensity corresponds to the retardance at each pixel location. Birefringence images can also be displayed as colored images, where different colors correspond to different values of retardance. Other display modalities can also be used to indicate differences in retardance from one pixel to another. Improved contrast can be used to highlight or emphasize particular structural elements of biological samples with respect to the rest of the sample.

The apparatus and systems include a sample manipulator that provides multiple degrees of freedom, *e.g.*, translation, rotation, to aid in positioning a sample. Specifically, the manipulator provides for rotation of a sample about an axis of the manipulator to orient birefringent structural elements within the sample with respect to an object plane of a birefringence imaging system.

Using a birefringence imaging system, a variety of different structural properties of a sample can be identified and quantified. Both qualitative observations and quantitative measures of structural properties can be used to derive metrics to describe sample properties. Metrics are calculated based upon birefringence images that are obtained in a systematic manner from an approximately uniform set of measurement conditions. As a result, the calculated metrics are reproducible and can be used for assessment purposes with a variety of samples, such as in a clinical practice like ART. For example, birefringence images of different samples that have one or more birefringent structural entities can vary according to the structure of the samples, and also according to the orientation of the birefringent structural entities within the samples with respect to a polarization state of illumination light used to obtain the images. Calculation of generally applicable metrics and comparison of images of multiple samples can therefore be facilitated if the images are recorded from samples which have been preferentially positioned and oriented so that the birefringent structural entities of interest in all samples are oriented in a common direction prior to obtaining the birefringence images. Methods, apparatus, and systems are disclosed herein that provide for positioning and orienting samples so that birefringent images of the samples can be recorded in a systematic, reproducible manner.

For example, consider the situation where a uniaxial, birefringent sample is imaged with its axis for extraordinary refraction aligned along the light propagation direction. Even though the sample may have a very large intrinsic birefringence (i.e., a large difference between *nₑ* and *nₒ*), little or no retardance will be observed in this situation because the incident light will see a largely isotropic index of refraction because the axis for extraordinary refraction lies along the propagation direction (and is therefore substantially orthogonal to the electric field vector for the light). On the other hand, consider a different sample having a smaller intrinsic birefringence, but which is aligned so that its axis for extraordinary refraction is substantially orthogonal to the light propagation direction (e.g., aligned in the object plane of the microscope). In this case, the observed retardance can be larger than that for the first sample, even though its intrinsic birefringence is smaller. The difference is that for the first sample the axis for extraordinary refraction is foreshortened when projected onto the object plane of microscope. In contrast, there is no such foreshortening for the second sample. This illustrates the importance of a systematic approach for orienting the sample with respect to the incident light. Absent such an approach, quantitative comparisons among measurements for different samples can be flawed because observed differences can be caused by differences in the way the samples are oriented, rather than by differences in sample properties.

By rotating the samples being measured in a systematic way, so as to orient their axes of extraordinary refraction to be substantially orthogonal to the propagation direction of the imaging light (*e.g.*, in the object plane of the microscope), one can maximize the observed retardance for each sample, and thereby facilitate quantitative comparisons between different measurements. Ideally, the axis for such rotation also lies in the object plane of the microscope, orthogonal to the light propagation direction. In such cases, there will always exist a rotation angle that will orient the extraordinary axis of the sample to lie in the object plane. However, even for rotation axes at small angles to the object plane (*e.g.*, angles of less than 30 degrees), the sample can be rotated to significantly reduce foreshortening of the extraordinary axis when projected onto the object plane, to allow quantitative comparisons between measurements for different samples. For example, for a rotation axis of about 18 degrees relative to the object plane, the maximum variation in observed retardance for optimally rotated samples having the same intrinsic birefringence is around +/- 5 percent.

Birefringence images of a sample and metrics derived from the images can be used to assess factors such as the developmental potential of a biological sample. For example, samples of mammalian oocytes can be evaluated with respect to the probability that each will lead to a healthy zygote upon fertilization. Particular structural features and elements of oocyte samples can act as indicators of the health of the oocyte, and images of these features and elements can be used to derive related quantitative metrics. These metrics can permit rapid, sensitive, quantitative, and non-destructive assessment of oocyte developmental potential in order to aid assisted reproductive technology therapies. Methods, apparatus, and systems are disclosed herein that provide for determining metrics from one or more sample images, and for determining the developmental potential of biological samples such as mammalian oocytes.

An important aspect of such a methods, apparatus, and systems is that because the birefringence images are obtained in a systematic, reproducible manner, quantitative comparisons between samples and/or with respect to reference information can be used to produce more accurate sample assessments. Such reference information can include reference values (e.g., threshold values), such as those that would be derived from quantitative metrics of one or more birefringence images of a reference sample taken under like conditions to those of the present sample of interest (such as optimal rotational orientation of the samples). Alternatively, or in addition, reference information can also include reference images of such reference samples, which may be used for comparison to make an assessment. As used herein, "quantitative metrics" are meant to include measures that are more detailed than simple binary information, such as the presence or absence of a structural entity in the image, but rather quantitative measures that provide or display a value or the like along some continuum of values (for example, length, size, retardance, distance, optical density, etc.). Nonetheless, in addition to such continuum values, quantitative metrics can, in certain embodiments, further include binary information.

Moreover, in certain embodiments, the methods and apparatus disclosed herein are applicable to the imaging of other types of samples, e.g., non-biological sample, and for imaging modalities different from birefringent imaging. For example, embodiments of the methods and apparatus described below provide for systematic alignment and orientation of imaging samples.

### I. Birefringence Imaging and Imaging Systems

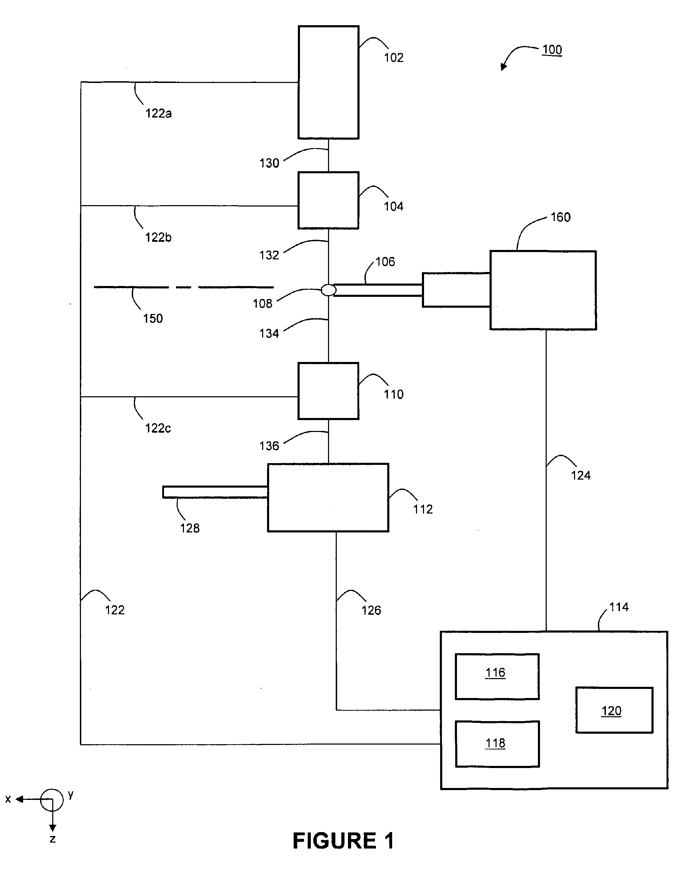

**FIG. 1** shows a schematic diagram of a birefringence imaging system **100** for obtaining birefringence images of a biological sample. System **100** includes an optical source **102** configured to provide source light **130.** Source light **130** passes through illumination optics **104** and is incident as illumination light 132 on sample **108.** A portion of illumination light **132** is transmitted through sample **108** as transmitted light **134,** and passes through detection optics **110.** Detection light **136** emerges from detection optics **110** and is incident on detector system **112.** A portion of detection light **136** can also be diverted by detection optics **110** so that it emerges from viewing port **128.** Detector system **112** is configured to capture images of sample **108,** which are then converted to electronic signals and conveyed to electronic control system **114** via communication line **126.** Electronic control system **114** includes a display device **116,** a user interface **118,** and a processor **120.** Electronic control system **114** can provide electronic control signals to detector system **112** through communication line **126,** and can also provide control signals to a sample manipulator **106** (details of which are described further below) via communication line **124.** Control signals can be provided to optical source **102,** illumination optics **104,** and detection optics **110** via communication lines **122a, 122b,** and **122c,** respectively.

Sample **108** is positioned in an object plane **150** of system **100.** With reference to the coordinate system shown in **FIG. 1,** object plane **150** is parallel to the *x*-*y* plane, and perpendicular to the *z* direction, which indicates the optical axis, or primary direction of propagation of source light **130.** Structures positioned in or near object plane **150,** such as sample **108** (or structural elements therein) are projected by system **100** onto an image plane (not shown). In some embodiments, more than one image plane may be present in system **100.** For example, in the embodiment shown in **FIG. 1,** detector system **112** is positioned in a first image plane of system **100,** and captures an image of sample **108** projected thereon. A second image plane of system **100** is located in viewing port **128,** whereon a second image of sample **108** is projected for viewing by a system operator. In general, other image planes can also be present in system **100,** and additional detectors and viewing ports can be provided in order to monitor and/or capture images of sample **108** projected onto those planes. As used herein, the "object plane" of the microscope is generally understood to be a plane within the depth of focus of the microscope that is substantially perpendicular to the optical axis of the microscope. The optical axis generally corresponds to the axis parallel to the propagation direction of the light being imaged through the system. Because the bundle of light rays being imaged typically span a range of propagation directions corresponding to numerical aperture of the microscope, the propagation direction is generally understood to be that of the center of the bundle (*e.g.*, the chief rays of the light ray bundle.)

In general, optical source **102** can be configured to provide source light **130** having a desired wavelength. For example, in some embodiments, source light **130** can have a wavelength in the ultraviolet, visible, or infrared portions of the electromagnetic spectrum. Source light **130** can include a narrow distribution of spectral wavelengths, *e.g.*, a full-width at half maximum of a spectral distribution of source light **130** can be about 0.5 nm or less. Alternatively, source light **130** can include, in some embodiments, a broader distribution of spectral wavelengths, *e.g.*, a full-width at half maximum of a spectral distribution of source light **130** can be larger than about 0.5 nm, such as 30nm or more. Optical source **102** can be configured to provide continuous-wave or pulsed source light **130** as desired for various imaging applications. Further, the intensity of source light **130** provided by optical source **102** can be selected based on one or more factors that can include brightness, contrast, and/or other properties of images of sample **108** obtained by detector system **112,** and the sensitivity of sample **108** to the intensity of illumination light **132.** In addition, optical source **102** can be configured to provide source light **130** in a desired polarization state. For applications such as birefringence imaging, source light **130** can be provided in a linear, circular, or elliptical polarization state, according to the manner in which the birefringence imaging technique is implemented.

Illumination optics **104** include optical elements configured to adjust spatial and/or spectral and/or polarization properties of source light **130.** For example, illumination optics **104** can be configured to cause convergence and/or divergence of source light **130.** As an example, illumination optics **104** can include one or more lens elements that collectively function as a condenser lens to project illumination light **132** upon sample **108.**

Filter elements in illumination optics **104** can be used to adjust the spectral properties of source light **130.** For example, in some embodiments, a spectral filter can be used to narrow or select the spectral width of source light **130.** In certain embodiments, a multiband filter or plural filters can be used to allow transmission of particular spectral sub-bands of source light **130.** Filters used in illumination optics **104** can have spectral bandpass shapes selected as desired to produce illumination light **132** having particular spectral band shapes.

Illumination optics **104** can also include polarization adjusting elements for changing the polarization state and/or orientation of source light **130.** For example, polarization adjusting elements can be used to interconvert source light **130** between linear, circular, and elliptical polarization states, the circular and elliptical polarization states having a desired left- or right-handedness. Further, the orientation of the polarization state of source light **130,** *e.g.*, the orientation of linearly polarized source light **130,** can be selected by suitably configuring polarization adjusting elements of illumination optics **104.**

Sample **108** is mounted on sample manipulator **106,** which will be discussed in more detail later. Sample manipulator **106** is attached to translation stage **160** which permits displacement of sample manipulator **106** in the *x, y,* and *z* directions. Thus, sample **108** can be re-positioned with respect to object plane **150,** *e.g.,* along the *z* direction, and also within any plane parallel to object plane **150.**

Detection optics **110** can include optical elements configured to provide functions that are similar to the functions provided by illumination optics **104.** That is, detection optics **110** can include elements that modify the spatial and/or spectral and/or polarization properties of transmitted light **134.** In some embodiments, for example, detection optics **110** can include elements configured to cause convergence and/or divergence of transmitted light **134,** and typically include one or more lens elements that function collectively as an objective lens. Detection optics **110** can also include elements for tailoring spectral properties of transmitted light **134,** and can further include elements for adjusting polarization properties of transmitted light **134,** including permitting interconversion of transmitted light **134** between linear, circular, and elliptical polarization states, and permitting adjustment of polarization orientation and handedness.

Detector system **112** is configured to capture one or more images of sample **108** based on a portion of source light **102** that is transmitted through sample **108** and is incident on detector system **112** as incident light **136.** Birefringence images of sample **108** can be obtained using a variety of different techniques and configurations of illumination optics **104** and detection optics **110.** In general, however, birefringence images are obtained wherein an intensity of detection light **136** at a selected pixel location (*x,y*) in an image corresponds to a measurement of optical retardance by sample **108** measured along the *z* direction and at transverse spatial positions (*x,y*) within sample **108.** Birefringence image data can be used to improve contrast or highlight certain structural features of sample **108** which may appear as lower contrast features in images obtained using other imaging techniques such as unpolarized broadband imaging. A suitable birefringence microscope is disclosed, for example, in commonly owned U.S. Patent No. 5,521,705 entitled "POLARIZED LIGHT MICROSCOPY," and U.S. Patent No. 6,924,893 entitled "ENHANCED POLARIZED LIGHT MICROSCOPY," referred to above and incorporated herein by reference.

The birefringence image data obtained by detector system **112** is conveyed to electronic processing system **114** via communication line **126** for further processing. Electronic processing system **114** can apply mathematical algorithms using processor **120** to transform the data and to calculate quantitative measurement metrics relating to sample **108** and its structural elements. Electronic processing system **114** can also process the data to generate images which are displayed on display device 116 to a system operator. The birefringence images of sample **108** can be grayscale images or colored images, *e.g.*, generated according to a mathematical algorithm that defines a color map. Measurements of optical retardance obtained from birefringence imaging techniques are quantitative, and therefore other algorithms can also be applied to the data to generate images for display purposes.

Some embodiments of birefringence images also provide information about the orientation of the extraordinary axis of birefringence (also termed the "slow" axis when *nₑ > nₒ)* of the material that comprises structures within sample **108.** In particular, when birefringence orientation information is available, one may examine how the birefringence is aligned relative to the shape of a structure, *i.e.* whether the slow axis of the material lies along a given direction or axis of a structure, or is transverse to it, or is oriented in some other way. This can give insight into how molecular orientation and/or composition within structures within sample **108.**

Quantitative measurements of optical retardance of sample **108** can be used for comparative and predictive purposes. For example, quantitative data can be used to calculate various metrics that compare selected structural features among various samples, and can also be used to make predictions (either by a system operator, or automatically by electronic control system **114)** regarding the developmental potential of the various samples. In some embodiments, a series of standardization steps are taken to ensure that samples are interrogated from a common set of measurement conditions, and to ensure that calculated metrics are derived from systematically measured, reproducible birefringence image data. In particular, birefringent structural entities in samples produce measured optical retardance values that are dependent upon both the birefringence properties of the structural entities, and the spatial orientation of the entities. In some samples, birefringence images of the same structural entity can yield different quantitative measurements of retardance if the orientation of the entity with respect to the optical axis of illumination light **132** is not taken into consideration. As a result, calculated metrics relating to a sample may not be reproducible, and may not be suitable for predictive purposes.

### II. Sample Handling and Manipulation

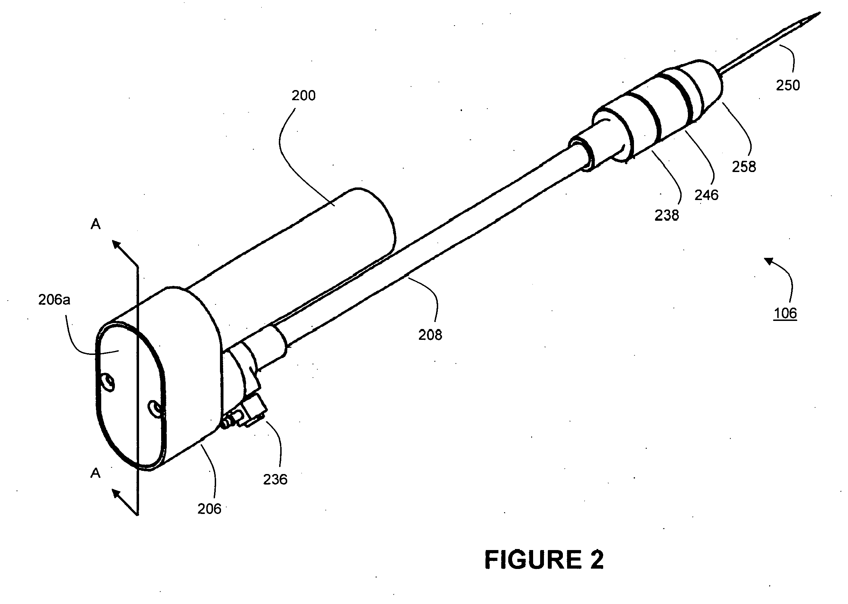

Orientational effects can be removed from birefringence imaging measurements by undertaking a series of steps to ensure that birefringent structural entities of interest are oriented in a preferred, common orientation prior to recording quantitative birefringence image data. One method for achieving a preferred, common orientation of structural entities of interest is to orient each sample **108** in a controlled manner, prior to obtaining the quantitative retardance measurement data used for assessment purposes. **FIG. 2** shows a perspective view of an embodiment of sample manipulator **106** that provides rotational degrees of freedom for orienting sample **108** in preferred orientations in order to establish systematic, reproducible initial conditions prior to recording quantitative optical retardance data. In general, sample manipulator **106** includes an extended hollow portion in the form of a pipette. As disclosed herein, a pipette is a hollow structure extending along a longitudinal axis and having openings at each of two ends. Typically, the pipette has one end narrower than the other end, where the size of the narrower end is sufficiently small to permit selective contact between the pipette and small objects. The pipette is mechanically coupled to a mount which can surround a portion of the pipette. In order to provide rotational degrees of freedom for orienting sample **108,** the mount is configured to permit the pipette to rotate with respect to its longitudinal axis. At the same time, the mount can maintain the pipette in a fixed position with respect to other degrees of freedom such as translational motion.

During use, sample **108** can be attached to one of the ends of the pipette. In many embodiments, attachment of sample **108** is by pressure differential - that is, by reducing a pressure of air inside the pipette so that a pressure of atmospheric air outside the pipette is greater than an air pressure inside the pipette. The resulting force on sample **108** positioned adjacent to an end of the pipette is sufficient to hold sample **108** in place against the pipette tip. Air pressure within the pipette is typically reduced by connecting a vacuum pump, squeeze bulb, or other pressure reducing device to an opening in the pipette, *e.g.*, the end opposite to the position of attachment of sample **108.**

To effect rotation of the pipette, an automated rotation mechanism such as a motor can be provided and connected to the pipette in order to enable rotation about the longitudinal axis. Alternatively, or in addition, some embodiments of sample manipulator **106** can permit manual rotation of the pipette about its longitudinal axis.

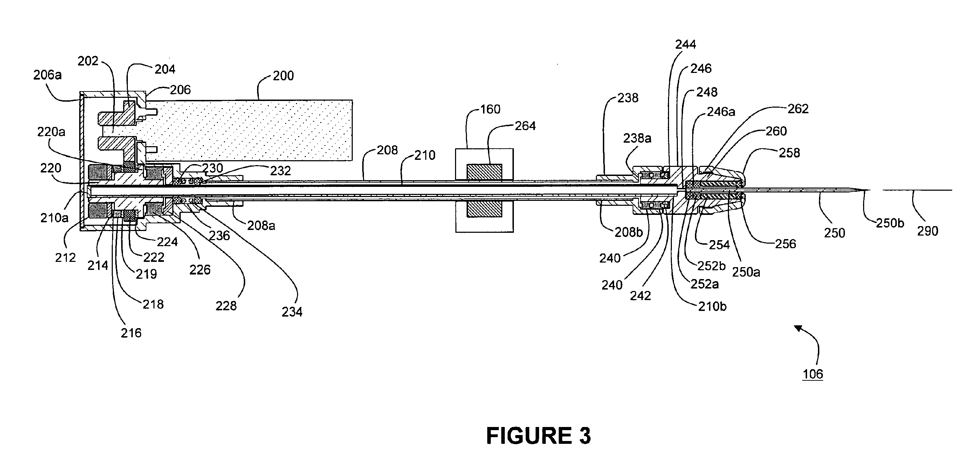

A cross-sectional view along line A-A of the sample manipulator **106** shown in perspective view in **FIG. 2** is shown in **FIG. 3.** Motor **200** has a rotating spindle **202** over which gear **204** is positioned. A locking screw (not shown) is used to ensure that motor spindle **202** and gear **204** rotate together when motor **200** is actuated. Gear **204** and spindle **202** can be fabricated from materials such as metals, *e.g.*, aluminum, steel, or from other materials such as a plastic material, *e.g.*, Delrin®.

Gear **204** is enclosed within housing **206.** Housing **206** is attached to outer tube **208** in region **208a** near one end of outer tube **208.** Attachment may be by means of epoxy deposited along a length of outer tube **208** that engages with housing **206.** Outer tube **208** can be fabricated from any of a variety of materials that can include glass materials, plastic materials, and metals. Outer tube **208** is extended along a longitudinal direction and has a longitudinal axis **290.** Positioned within outer tube **208** and having a collinear longitudinal axis **290,** inner tube **210** can also be fabricated from any of a variety of materials that can include glass materials, metal materials, and plastic materials. While outer tube **208** is rotationally fixed in position relative to housing **206** due to epoxy bonds in region **208a,** inner tube **210** can rotate about longitudinal axis **290** relative to housing **206** either in response to a manual torque applied by a system operator, or in response to a torque applied to spindle **202** by motor **200.** In the present embodiment, an end portion **210a** of inner tube **210** is sealed to ensure that a reduced pressure environment can be maintained within inner tube **210.**

Rear panel **206a** can be removed from housing **206** to provide access to the rear portion of sample manipulator **106** for assembly and repair, as needed. Arbor **220** is positioned over end portion **210a** of inner tube **210** and is held in place by locking clamp **212,** which applies a radial force to arbor **220** to ensure that arbor **220** and inner tube **210** rotate together about axis **290** by introducing a sufficiently high coefficient of static friction between these elements. Both arbor **220** and locking clamp **212** can be fabricated from metals, or from plastic materials such as Delrin®, for example.

Hubless gear **222** is positioned around arbor **220** so that some of the teeth of hubless gear **222** are in engagement with some of the teeth of gear **204.** On one side of hubless gear **222,** clutch face **224** is positioned between flange **220a** of arbor **220** and hubless gear **222.** Clutch face **224** is fabricated from a material such as metal or plastic, *e.g.*, Delrin®. On the other side of hubless gear **222,** clutch **218** is spaced from hubless gear **222** by clutch spacer **219,** and clutch **218** and clutch spacer **219** are positioned tightly against hubless gear **222** by wave spring **216.** Clutch **218** is fabricated from a material such as cork. Clutch spacer **219** can be fabricated from metal materials, or from plastic materials such as Delrin®. Wave spring **216** includes a compressible material that applies a force in a direction parallel to axis **290** which ensures that clutch **218** and hubless gear **222** fit tightly together. The force applied by wave spring **216** can be changed by selecting a different material for the spring, or a different spring size. Spacer **214** separates wave spring **216** from locking clamp **212,** and can be fabricated from metal materials, or from plastic materials such as Delrin®.

Bearing **226** is positioned between inner tube **210** and housing **206.** Bearing **226** permits smooth, low-friction rotation of inner tube **210** about axis **290** while housing **206** remains rotationally fixed in position. Simultaneously, bearing **226** permits housing **206** to support inner tube **210** so that, for example, inner tube does not wobble or undergo translational motion with respect to housing **206** as inner tube **210** is rotated about axis **290.**

Access port **236** is positioned adjacent to bearings **226.** First O-ring **230** separates a first sub-chamber of housing **206** containing arbor **220** from a second sub-chamber containing access port **236,** and maintains an air tight seal between the two sub-chambers. Second O-ring **232** is spaced apart from first O-ring **230** by spacer **234** and separates the second sub-chamber from a third sub-chamber of housing **206** where outer tube **208** is attached with epoxy, and ensures an air tight seal between the second and third sub-chambers. Thus, access port **236** is isolated from the rest of the interior of housing **206** by O-rings **230** and **232,** and provides a connection port for a vacuum pump or for another pressure reducing device. Spacer **234** can be fabricated, for example, from metal materials, plastic materials (*e.g.*, Delrin®), or other materials.

In the vicinity of access port **236** and within the second sub-chamber of housing **206,** a small hole (not shown) is provided in inner tube **210.** When a vacuum pump is connected to access port **236,** air pressure in the second sub-chamber of housing **206** is reduced. Since inner tube **210** is in fluid connection with the second sub-chamber via the small hole, air pressure inside inner tube **210** is also reduced.

The other end **208b** of outer tube **208** is attached to bearing holder **238** using epoxy applied between the mating surfaces of these elements. Inner tube **210** extends through bearing holder **238** and an end **210b** of inner tube **210** is attached to pipette holder **246** via epoxy applied to mating surfaces of inner tube **210** and pipette holder **246.** Inner tube **210** and pipette holder **246** are rotatable about axis **290** relative to outer tube **208** and bearing holder **238** by means of bearings **240** positioned between bearing holder **238** and pipette holder **246.** Bearings **240** provide for smooth, low-friction rotation of inner tube **210** relative to bearing holder **238,** and also ensure that bearing holder **238** and pipette holder **246** do not wobble or undergo substantial translational motion with respect to one another as inner tube **210** rotates about axis **290.** Bearing holder **238** and pipette holder **246** can be fabricated from metals, *e.g.*, aluminum or steel, or from plastic materials such as Delrin®, for example.

Bearings **240** are positioned to fit against flange **238a** of bearing holder **238** on one side, and against spacer **242** on the other side. Spacer **242** can be fabricated from a metal material, or from a plastic material such as Delrin®, for example. Wave spring **244,** fabricated from a compressible material, applies a force in a direction parallel to axis **290** to ensure that bearing holder **238,** bearings **240,** and spacer **242** fit tightly against one another. The force applied by wave spring **244** can be changed simply by selecting a different wave spring size or material.

End **210b** of inner tube **210** remains in an open configuration and is positioned within pipette holder **246** adjacent to port **248.** Port **248** is therefore in fluid connection with inner tube **210** and a region of reduced pressure within inner tube **210** extends into port **248.**

On the other side of port **248,** a pipette **250** is positioned so that a longitudinal axis of pipette **250** is collinear with axis **290.** End **250a** of pipette **250** is open, so that pipette **250** is in fluid connection with port **248** and inner tube **210.** As a result, the region of reduced air pressure extends into pipette **250** from end **250a** to end **250b.**

Pipette **250** is maintained in secure engagement with an end of port **248** by a clamping mechanism. O-rings **252a** and **252b** are positioned within a recess **246a** of pipette holder **246** and about a circumference of pipette **250.** A spacer **254** made from a material such as Delrin® is adjacent to the O-rings, and another O-ring **256** is positioned adjacent to the opposite side of spacer **254.** Cap **258** encloses recess **246a** of pipette holder **246,** O-rings **252a, 252b,** and **256,** and spacer **254.** Threads **260** on an outer surface of pipette holder **246** engage with grooves **262** on an inner surface of cap **258.** By applying a rotational torque to cap **258,** cap **258** can be screwed into secure engagement with pipette holder **246,** applying compressive force in a direction parallel to axis **290** to O-rings **252a, 252b,** and **256,** and to spacer **254.** In response to the applied force, the O-rings deform and are compressed into intimate contact with pipette 250, ensuring an air-tight connection between access port **248** and the interior of pipette **250,** and fixing pipette **250** in position relative to inner tube **210.**

Inner tube **210** can be rotated by actuating motor **200.** Motor **200** applies a torque to spindle **202,** causing spindle **202** to rotate about an axis parallel to axis **290.** The rotational motion of spindle **202** is communicated to inner tube **210** via gear **204** in engagement with hubless gear **222.** As a result, rotational motion of inner tube **210** about axis **290** is initiated in response to torque applied by motor **200** to spindle **202.** Thus, a rotational orientation of a sample positioned at end **250b** of pipette **250** can be selected automatically by actuating motor **200.** In some embodiments, the rotational orientation of the sample can also be adjusted by manually rotating pipette holder **246,** *e.g.*, by manually applying a torque to pipette holder **246.**

Sample manipulator **106** is configured to prevent manual rotation of inner tube **210** from causing damage to the motor **200** or other drive-train components. If inner tube **210** is rotated by manually applying a torque to pipette holder **246,** then the applied torque will be communicated to spindle **202** via gear **204** in engagement with hubless gear **222.** Spindle **202** will resist undergoing rotational motion. As the manually applied torque increases, and before damage to motor **200** occurs, hubless gear **222** will slip relative to clutch **218,** allowing inner tube **210** to rotate without inducing concurrent rotation of spindle **202,** and thereby preventing damage to motor **200.** Appropriate selection of the material of clutch **218** and of wave spring **216** according to a coefficient of static friction between clutch **218** and hubless gear **222** can be used to set a threshold for slipping of hubless gear relative to clutch **218** under an applied torque to inner tube **210.**

Translational motion of sample manipulator **106** can be effected using translation stage **160.** A mounting attachment **264** is provided that connects to a portion of outer tube **208** and is affixed to translation stage **160.** Translation stage **160** provides for translation of manipulator **106** in each of three independent coordinate directions. End **250b** of pipette **250,** along with an attached sample, can therefore be positioned at any three-dimensional coordinate location within a range of travel of translation stage **160,** relative to a selected reference position. Further, a rotational orientation of the attached sample can be selected by actuating motor **200** to rotate inner tube **210.** Because inner tube **210** and pipette **250** are mechanically coupled through pipette holder **246,** a rotational orientation of the attached sample can be adjusted. Outer tube **208,** which is bonded to housing **206** and bearing holder **238,** does not rotate in response to actuation of motor **200.**

Pipette **250** can be a commercially available glass pipette. Suitable pipettes can be obtained, for example, from Cook Vascular Inc. (Leechburg, PA). Typically, the tip of pipette **250** has an outer diameter between about 90 microns and about 120 microns, and an inner diameter between about 15 microns and about 50 microns, although other sizes for both the inner and outer diameters are also possible. In particular, the inner diameter of pipette **250** can be selected in view of a size of a sample of interest, where the inner diameter can be chosen according to the usual criteria for pipette selection, such as to be slightly smaller than the size of the sample. In some embodiments, inner tube **210** can be used repeatedly for multiple samples, and pipette **250** can be disposable, and can be replaced after every sample.

Not all pipettes **250** are straight along longitudinal axis **290,** however. In particular, some pipettes, including some commercially available pipettes, can deviate from an ideally straight geometry along axis **290** by about 50 microns or more. As a result, rotation of pipette **250** to select a new orientation for a sample attached to end **250b** of pipette **250** can lead to a translational displacement of the sample. Translational displacements can be compensated by applying rotation-dependent corrective translations via translation stage **160,** as will be described in greater detail later.

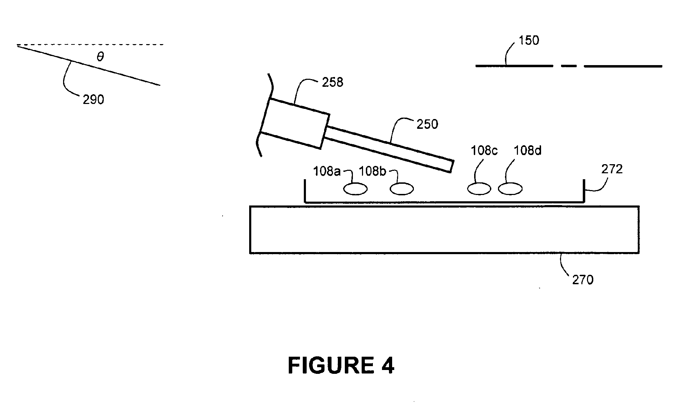

In use, a selected sample is attached to end **250b** of pipette **250. FIG. 4** is a schematic diagram showing positioning of a sample relative to pipette **250.** One or more samples, shown schematically as samples **108a-d,** are typically provided in a sample dish **272** positioned on a support stage **270** of system **100.** Dish **272** is supported by support stage **270** so that a bottom surface of dish **272** is oriented substantially parallel to object plane **150** of system **100.** A position of pipette **250** is adjusted so that end **250b** of pipette **250** is positioned in proximity to one of the samples. Air pressure in pipette **250** is then reduced by connecting a vacuum pump or other pressure-reducing device to access port **236.** Subsequently, the position of pipette **250** is adjusted in small increments so that end **250b** makes gentle contact with one of the samples **108a-d.** Due to an air pressure differential between atmospheric air and the interior of pipette **250,** the selected sample is attached to end **250b** of pipette **250** and held in place. Pipette **250** can then be translated away from dish **272,** *e.g.*, in a direction orthogonal to object plane **150.** The various translational displacements described in connection with attaching a sample to and end of pipette **250** can, in general, be accomplished either by manual manipulation of translation stage **160,** or by operating translation stage **160** in automated fashion, *e.g.*, by using a series of motors or actuators to provide the desired translations.

As shown in **FIG. 4,** sample dish **272** can have sides which prevent longitudinal axis **290** of pipette **250** from being oriented strictly parallel to object plane **150.** In particular, pipette **250** can be oriented at a specific mounting angle θ with respect to object plane **150.** The value of θ depends upon the height of the sides of dish **272** and other geometrical parameters of system **100,** and is typically in a range from about 5 degrees to about 30 degrees. In one useful arrangement, the angle θ is approximately 18 degrees. However, the mounting angle can generally vary as desired in order to accommodate specific dishes or other containers in which a sample is provided.

### III. Obtaining Calibrated Birefringence Images

Once a sample **108** is attached to sample manipulator **106,** the coordinate position of sample **108** can be adjusted relative to object plane **150** of system **100** to improve measurement reproducibility and/or resolution of structures in birefringence images obtained using the system.

Rotation of sample **108** about axis **290** can also provide improvements in measurement reproducibility and/or resolution of structures in images of a variety of different sample types. In particular, this may be true for birefringent imaging or other types of imaging such as fluorescence imaging. For samples that include birefringent structural entities, contrast between these entities and the remaining (non-birefringent) portions of sample **108** may depend on the spatial orientation of the birefringent entities. In fluorescence imaging, structures may appear brighter or clearer if oriented in a preferred orientation, or alternatively may be dimmer or obscured in other orientations. Therefore, for certain samples, image contrast between different portions of sample **108** can be improved by selectively changing the orientation of sample **108** by effecting rotation of pipette **250** about axis **290.** For example, a particular type of sample that includes birefringent structural entities is a mammalian oocyte. As will be described in further detail later, mammalian oocytes contain structures such as spindles that exhibit birefringence. Adjustment of the orientation of a sample oocyte can be used to increase contrast in birefringence images showing the spindles.

In general, better resolution in birefringence images of sample **108** is obtained when sample **108** is positioned in or close to object plane **150.** However, rotation of sample **108** about axis **290** can displace sample **108** from object plane **150** due to deviations from straightness of pipette **250** along axis **290.** Further, rotation of sample **108** about axis **290** can displace sample **108** within object plane **150,** so that images of sample **108** obtained at different rotation settings do not overlap spatially with one another.

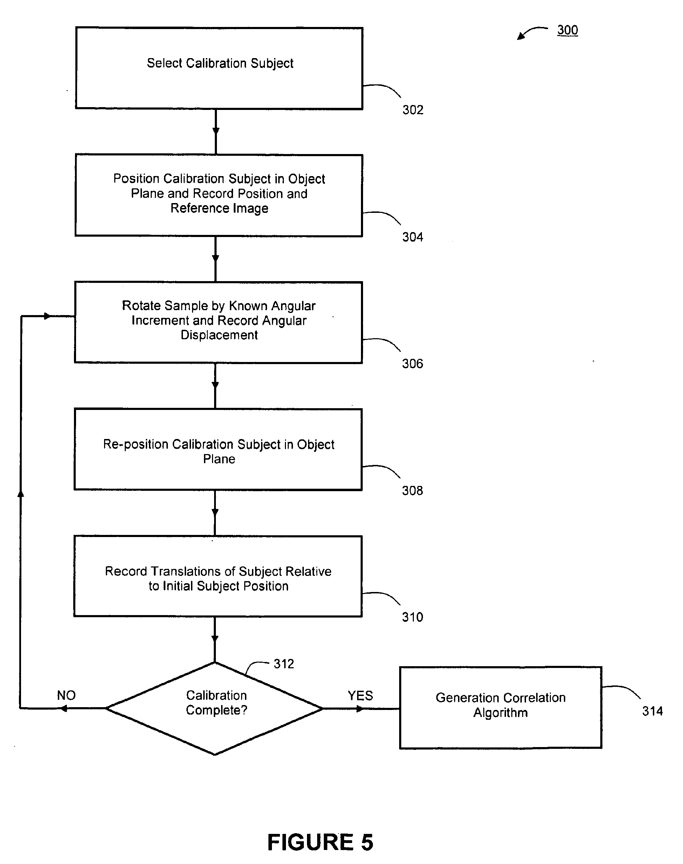

Sample manipulator **106** can be calibrated in order to compensate for rotation-dependent translation of a sample attached to pipette **250.** In general, a new calibration of sample manipulator **106** may be performed each time a new pipette **250** is introduced, since each new pipette is expected to deviate by a different amount from ideal linearity along axis **290. FIG. 5** is a flow chart **300** that shows steps involved in calibrating sample manipulator **106** to correct for curvature in pipette **250.** In a first step **302,** a calibration subject is selected. Various subjects can be selected to provide calibration data during the calibration process. For example, a sample such as an oocyte can be mounted on pipette **250** and imaged to provide calibration data. Alternatively, a glass or plastic bead can be mounted and used to provide the calibration data. As another alternative, a structural feature such as a minor imperfection on end **250b** of pipette **250** or the pipette tip itself can be imaged using system **100** to provide data for the calibration process. In general, the calibration subject may or may not be birefringent, and may or may not be a sample of interest.

Having selected a suitable calibration subject, the subject is translated in second step **304** so that the subject is positioned in object plane **150,** and at a suitable location within a field of view of system **100** in object plane **150.** Positioning of the calibration subject with respect to object plane **150** is generally determined by a system operator or automatically by software within electronic control system **114,** based on images of the calibration subject recorded as the subject is translated in directions perpendicular and parallel to object plane **150.** In general, images of the calibration subject will have a sharper focus when the calibration subject is positioned in object plane **150.** If the calibration subject is a feature on end **250b** of pipette **250,** for example, details of the feature will be mostly clearly resolved when the feature is located in object plane **150.** If the calibration subject is a sample, *e.g.*, an oocyte, or a bead, then images of the sample will have a maximum cross-sectional sample area when the sample is positioned in object plane **150.** In addition, edges of the sample will be most clearly resolved in images when the sample is positioned in object plane **150.** Using these qualitative criteria, the calibration subject is positioned in object plane **150** and a reference image is recorded, along with the position (*x, y, z* coordinate) settings of translation stage **160.** An axial rotation angle of pipette **250** about axis **290** is assigned a value of zero degrees.

In step **306,** the axial rotation angle of pipette **250** is changed by a known increment, *e.g.*, an increment of about 2-30 degrees, for example. If pipette **250** is not straight, or is not perfectly collinear with axis **290,** rotation of pipette **250** about axis **290** will displace the calibration subject along some or all of the *x, y,* and *z* coordinate directions. The axial rotation angle of pipette **250** is recorded and stored in a calibration table.

In step **308,** the calibration subject is translated along some or all of the coordinate axes using translation stage **160** in order to re-position the calibration subject within object plane **150.** For example, the calibration subject may be first translated in a direction perpendicular to object plane **150** until the calibration subject is positioned within object plane **150,** as determined using the criteria discussed above. Subsequently, the calibration subject can be re-positioned within object plane **150** so that an image of the calibration subject in cross-section is spatially coincident with the reference image of the calibration subject recorded in step **304.** The spatial overlap between the two images can be assessed by a system operator, for example, or a spatial overlap metric can be calculated by electronic control system **114** in order to quantify the spatial overlap between the images.

When the calibration subject is appropriately positioned with respect to object plane **150,** the relative translations along each of the coordinate directions (*e.g.*, relative to the position settings recorded in step **304)** are recorded and stored in step **310,** with reference to the rotation angle recorded in step **306.** Step **312** then introduces a logical decision. If a complete set of calibration data has been acquired, control passes to step **314.** Alternatively, if a complete set of calibration data has not been acquired, control reverts to step **306,** where pipette **250** is rotated by another angular increment and the calibration subject is re-positioned.

In some embodiments, a complete set of calibration data for a pipette **250** can be acquired by rotating pipette **250** through a complete axial revolution. Alternatively, in some embodiments, a complete set of calibration data can be acquired for a range of axial rotation angles that is less than 360 degrees. For example, due to cylindrical rotation symmetry of sample manipulator **206** about axis **290,** a set of calibration data acquired over a smaller range of axial rotation angles (*e.g.*, a 180 degree range of angles, a 90 degree range of angles) can be used to infer corrective rotation-dependent translations of pipette **250** at other axial rotation angle settings of pipette **250.**

Once the calibration data has been collected, a correlation algorithm relating the axial rotation angle to suitable corrective three-dimensional translational displacements for pipette **250** can be generated. The correlation algorithm provides a mechanism to determine, for a particular axial rotation angle, an appropriate set of translational displacements that, when applied, ensure that a sample remains in a selected position with respect to object plane **150.** The correlation algorithm can be a calibration table for example, where translational displacements for particular axial rotation angles not appearing in the table are interpolated mathematically. Alternatively, the calibration data can be fitted to a set of mathematical functions that describe translational displacements in each of the three coordinate directions *x, y,* and *z* as a function of axial rotation angle, so that a particular set of translational displacements can be calculated for any selected axial rotation angle. In birefringence imaging applications, when sample **108** is rotated about axis **290,** corrective translational displacements can be automatically introduced via translation stage **160** to ensure reproducible positioning of sample **108** relative to object plane **150.**

The calibration steps described above can be performed manually by a system operator, or some (or all) of the steps can be performed automatically by electronic control system **114,** for example. Software running on processor **120** can be programmed to execute the logic steps shown in flow chart **300** in order to implement the calibration methods. Further, translational and/or rotational displacements of pipette **250** can be effected automatically by electronic control system **114** via control signals transmitted to sample manipulator **106** through communication line **124.**

In some embodiments, birefringence imaging techniques are used to obtain calibration data. For example, when the calibration subject is a birefringent sample such as a mammalian oocyte, birefringence images may provide better image contrast near the edges of the calibration subject, which may assist in determining when the subject is appropriately positioned in object plane **150.** In some embodiments, a different imaging modality such as bright-field transmitted light, differential interference contrast, relief contrast, or polarized light imaging can be used to determine when the calibration subject is appropriately positioned in object plane **150.**

The disclosed methods permit systematic acquisition of birefringence images of different samples under controlled, reproducible conditions using system **100.** Quantitative analysis of the birefringence images can yield reproducible metrics for sample assessment. In the following section, applications of these calibrated birefringence imaging techniques to specific types of samples will be discussed.

### IV. Oocyte Structural Features

Mammalian oocytes form a class of birefringent samples that are of interest with regard to improving the efficiency of assisted reproductive technologies used in human and animal therapies. For example, fertilization of oocytes having a high likelihood of producing healthy offspring can improve the efficiency and lower the cost of ART therapies.

An oocyte is an unfertilized female gamete (sex cell). Mature gametes are haploid cells - that is, they have only half the number of chromosomes found in all other cells in the body. Fertilization, or fusion of the male and female gametes, results in a cell with exactly the full (or diploid) chromosome complement for normal, healthy development. The specialized form of cell division that gives rise to gametes is called meiosis, a highly regulated, exquisitely timed, multi-step process of two sequential cell divisions (meiosis I and meiosis II) that ensure that the mature gametes have the usual haploid number of chromosomes. The successive stages of these meiosis processes (interphase I, prophase I, metaphase I, anaphase I, and telophase I, followed by prophase II, metaphase II, anaphase II and telophase II) are defined based on the organization and location of the chromosomes in the oocyte. Meiotic cell divisions in oocytes are highly asymmetric, resulting in the formation of large daughter oocytes and smaller cells called polar bodies. A detailed description of meiosis can be found in standard biology textbooks such as, for example, Campbell, Neil A. and Reece, Jane B, *Biology* (Benjamin Cummings, Menlo Park, CA, 2001).

Oocytes can be characterized as primary or secondary oocytes depending upon their stage of development and position in transit through meiosis. As used herein, the term "oocyte" refers to both primary and secondary oocytes. Oocyte development begins early in fetal life when primordial germ cells, the precursors to mature gametes, migrate into gonadal ridges of the developing embryo. The primordial germ cells proliferate within ovarian follicles and differentiate into primary oocytes, which arrest at prophase of the first meiotic division (*e.g.*, prophase I). This period of arrested development in primary oocytes can extend for many years. In humans, for example, these cells number a few hundred thousand at birth and represent the entire complement of oocytes a woman will have in her lifetime. More than 99% of these primary oocytes never ovulate, but undergo atresia within the ovary or apoptosis before or after fertilization.

In the presence of specific hormonal stimuli, a primary oocyte completes the first meiotic division and progresses through to metaphase of the second meiotic division (*e.g.*, metaphase II), generating a large cell called a secondary oocyte and a much smaller cell called a first polar body. At the same time, the ovarian follicle ruptures and the secondary oocyte is released into the fallopian tube to begin its journey to the uterus. The secondary oocyte can also be referred to as a metaphase II (MII) oocyte, or alternatively, an ovum, or an egg. Fertilization, or union of the sperm and the egg, takes place within the fallopian tube and triggers a complex series of events including completion of the second meiotic division, extrusion of a second polar body, and formation and fusion of the male and female pronuclei. The fertilized oocyte (which can also be referred to as a zygote, or fertilized egg) therefore has a diploid number of chromosomes that provide the fertilized oocyte with the potential to develop into an independent organism.

Following fertilization, the zygote moves through the fallopian tube toward the uterus. At the same time, it undergoes a series of rapid cell divisions with little increase in its absolute size, cleaving to 2, then 4, then 8, then 16, then 32, and then 64 cells. As cleavage continues, the zygote begins to resemble a ball of cells. Between the 4 and 16 cell stage (also known as the morula stage) the cells on the inside of the ball and the cells on the outside of the ball begin to differentiate and take on different functional characteristics. The outer cells pump fluid from the surrounding medium into the center of the zygote to produce a fluid-filled central cavity termed the blastocoel. By the time the zygote includes about 64 cells, at the blastocyst stage, the zygote is composed of two distinct populations of cells. Attached at one side of the inner wall of the blastocoel is a clump of cells called the inner cell mass that gives rise to the embryo. The cells on the outside of the blastocyst are called the trophoblast. The trophoblast cells function to implant the blastocyst in the epithelium of the uterus, where the inner cell mass further differentiates into primitive ectoderm and the remainder of prenatal development occurs. As used herein, a "fertilized oocyte" refers to an oocyte at any stage of development between the union of the sperm and the egg, and inner cell mass differentiation.

"Developmental potential", as used herein, refers to sequential acquisition by the oocyte of both meiotic and developmental competence, (*e.g.*, the ability of the primary oocyte to resume and complete meiosis, and the ability of the secondary, metaphase-arrested oocyte to be fertilized and support development to term, respectively.)

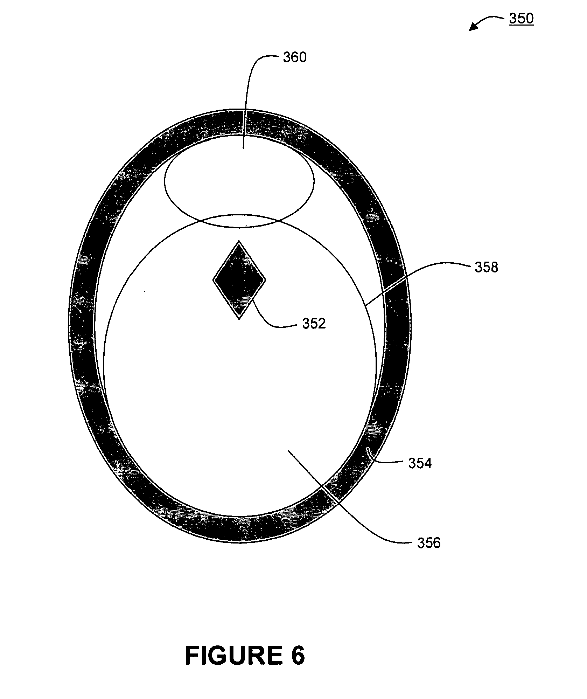

A schematic diagram of a mammalian oocyte **350** is shown in **FIG. 6.** Oocyte **350** is a secondary or MII oocyte, although many of the structural elements shown are also present in other types of oocytes. However, not all of the structural elements shown are present at all stages of oocyte development. For example, the meiotic spindle is a transiently formed structure that is present only at certain stages of meiosis, while the zona pellucida is present throughout oocyte development and after fertilization. The structure elements shown in **FIG. 6** are described in further detail below.

### A. The Meiotic Spindle

The meiotic spindle **352** is a cellular organelle that organizes and moves chromosomes during meiosis. Meiotic spindle **352** is composed of microtubules and other associated proteins such as kinesins and dyneins. Microtubules are highly organized, naturally birefringent fibers found throughout the cell that serve as tracks along which other cellular organelles can move. The microtubules that form the meiotic spindle are arranged in parallel, with their ends joined together and their midsections widely spread to form a structure with a "barrel" or "football-shaped" appearance. In human oocytes, the spindle is a dynamic structure that begins to assemble during the prophase stage of meiosis, separates and moves the chromosomes to proper destinations in oocyte **350** and polar body **360** during metaphase, and finally disassembles as meiosis progresses. The spindle is located near the oocyte cell membrane or oolemma **358** (see below) with a long axis of the spindle fibers extending approximately parallel to a diameter of oocyte **350** and perpendicular to oolemma **358.**

### B. The Zona Pellucida

The zona pellucida **354** is an extracellular glycoprotein matrix surrounding the plasma membrane of oocyte **350.** The zona pellucida **354** (or simply the "zona") has diverse functions depending upon the developmental stage of the oocyte. In an unfertilized oocyte, zona **354** binds and activates sperm during fertilization and then provides a block to polyspermy, *e.g.*, fertilization of the egg by more than one sperm. After fertilization, zona **354** encapsulates the zygote to prevent disaggregation of the uncompacted embryonic cells, to block premature attachment of the embryo to an oviduct surface, and to protect the embryo from toxins, bacteria, viruses and maternal phagocytes. Once the embryo reaches the uterus, it "hatches" from zona **354** and implants in the uterine endometrium.

Zona **354** is composed of several glycoproteins that are arranged in a highly organized fashion that results in natural birefringence. In some species, *e.g.*, humans and rats, zona **354** includes four glycoproteins, ZP1, ZP2, ZP3, and ZP4. In other species, *e.g.*, mice, zona **354** lacks ZP4. When viewed under polarized light, zona **354** appears as a trilaminar structure composed of two highly birefringent layers separated by an intermediate layer. The filaments of the innermost layer are oriented radially relative to oocyte **350** while those in the outermost layer are oriented tangentially relative to oocyte **350.**

### C. The Cytoplasm

The cytoplasm **356** of oocyte **350** includes the entire contents of the cell, exclusive of the nucleus, bounded by oolemma **358.** The nucleus, which contains the chromosomes and associated proteins within a network of fibers known as the nuclear matrix, is partitioned from cytoplasm **356** by a specialized membrane called the nuclear envelope. Cytoplasm **356** includes subcellular organelles required for cell functioning suspended in the cytosol, a semi-fluid medium. The subcellular organelles can include: organelles involved in biosynthesis of proteins and other essential molecules, *e.g.*, ribosomes, smooth and rough endoplasmic reticulum; organelles involved in intracellular transport, *e.g.,* Golgi apparatus, and microtubules; organelles involved in energy transformation, *e.g.,* mitochondria; and organelles involved in structural support, *e.g.*, the cytoskeleton. The composition of cytoplasm **356** changes as oocyte **350** development progresses. A primary oocyte has a relatively large nucleus called a germinal vesicle. A germinal vesicle membrane breaks down as the first meiotic division proceeds. A secondary oocyte lacks a nucleus and a nuclear envelope is reformed only after the secondary oocyte is fertilized. Thus, the cytoplasm in a primary oocyte and in a fertilized oocyte is partitioned from the nucleus, whereas the cytoplasm in a secondary oocyte is not, and will include molecules that typically are restricted to the nucleus in primary oocyte and fertilized secondary oocytes.

### D. The Oolemma

Cytoplasm **356** of oocyte **350** is enveloped by a specialized plasma membrane called the oolemma **358.** Plasma membranes or cell membranes are composed of a fluid bilayer of phospholipids and protein molecules. Typically, oolemma **358** functions to control the movement of molecules in and out of the cell, interactions between cells, and interactions between cells and acellular matrices. In addition to standard membrane components, oolemma **358** also includes specialized molecules involved in sperm-egg fusion. Although the molecular mechanism of fertilization has not yet been clearly elucidated, it is known that the plasma membrane surrounding the sperm binds to oolemma **358** and the two plasma membranes fuse.

### E. The Polar Body

The polar body **360** is a small cell that contains excess chromosomes discarded during meiosis. Two polar bodies are typically generated during meiosis in mammals. First and second polar bodies result from first and second meiotic divisions, respectively. Polar body **360** lies in the perivitelline space, that is, the space between oolemma **358** and zona pellucida **354.**

### V. Systematic Alignment of Oocyte Samples for Birefringent Imaging

Oocyte **350** is approximately spherical in shape, although there is wide variation in absolute size depending upon both the organism involved and the stage of development of oocyte **350.** An "equatorial plane" of oocyte **350,** as used herein, refers to a cross-sectional plane of oocyte **350** which divides the oocyte into two approximately equal hemispherical halves according to volume. When an equatorial plane of oocyte **350** is positioned in an object plane of an imaging system, the cross-sectional area of an image of the oocyte is typically a local maximum.

Due to the approximately spherical shape of oocyte **350,** a cross-sectional view of oocyte **350** that corresponds to an equatorial plane is approximately circular in shape. In humans, a diameter of oocyte **350** in an equatorial cross-sectional view, measured between outer surfaces of zona pellucida **354,** is typically in a range from about 70 microns to about 190 microns.

Meiotic spindles are of particular interest with respect to assessing the developmental potential of an oocyte. Meiotic spindles are birefringent structural elements, with birefringent axes located along the long and short polar axes of the spindles. Absent any qualifiers to the contrary, the "polar axis" of the spindle will be understood to mean the long polar axis of the spindle. In birefringence images of a meiotic spindle, a measured retardance due to the spindle is determined both by the structure of the spindle and by its three-dimensional orientation with respect to object plane **150b** of measurement system **100.** In order to measure birefringence images of oocytes and calculate oocyte metrics in a systematic, reproducible manner, the orientation and position of each oocyte are adjusted prior to obtaining quantitative oocyte birefringence data. Adjustment of an oocyte's position includes translating the oocyte in a direction perpendicular to object plane **150** of system **100** to position the oocyte in object plane **150.** Adjustment of the oocyte's orientation includes rotation of the oocyte about axis **290** of sample manipulator **106** to ensure that the polar axis of the oocyte's meiotic spindle lies in, or nearly in, object plane **150.** For example, we consider the polar axis of the spindle to lie substantially in the object plane when it forms angle of less than 30 degrees, and more preferably less than 20 degrees with the object plane. Subsequently, images of the oocyte are obtained using birefringence imaging system **100,** and used for comparative or assessment purposes.

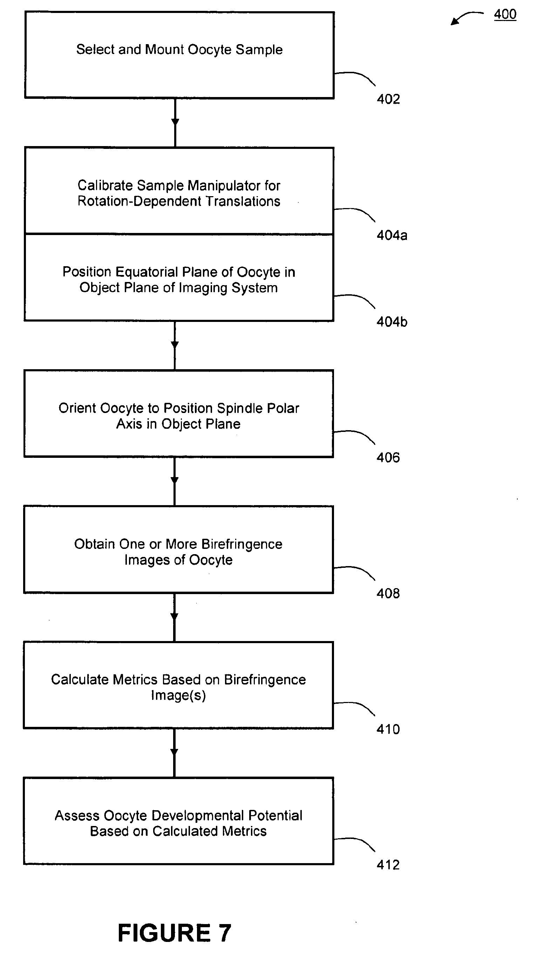

A sequence of steps involved in obtaining oocyte images and assessing an oocyte's developmental potential is shown in flow chart **400** of **FIG. 7.** This is preferably done via the sample manipulator apparatus described above, though in the alternative one can make use manual methods such as manipulation described below. In the preferred embodiment, step **402** includes selecting and mounting an oocyte on end **250b** of pipette **250,** as described in connection with **FIG. 4.** Step **404a** is an optional calibration step that can be performed in order to correct sample manipulator **106** for rotation-dependent translation of the sample oocyte. If step **404a** is performed, then an equatorial plane of the oocyte is positioned within object plane **150** of system **100,** and control passes to step **406.** If step **404a** is not performed, step **404b** is executed and the sample oocyte is translated in a direction perpendicular to object plane **150** until an equatorial plane of the oocyte is located in object plane **150.** Suitable criteria for determining when an equatorial plane of the oocyte is located in object plane **150b** can include, for example, determining a position of the oocyte that yields a birefringence image in which a demarcation between the cytoplasm and the zona pellucida is sharpest. Alternatively, or in addition, suitable criteria for determining when an equatorial plane of the oocyte is located in object plane **150b** can include determining a position of the oocyte that yields a maximum cross-sectional area of the oocyte in a set of birefringence images.

After the equatorial plane of the oocyte is positioned within the object plane **150,** the oocyte is re-oriented in step **406** to position the polar axis of the oocyte's meiotic spindle as nearly as possible within object plane **150,** while the equatorial plane of the oocyte is maintained in object plane **150.** Criteria for determining whether the polar axis of the spindle is in, or as nearly aligned as possible, to object plane **150** can be an assessment of spindle focus in each of a set of images by a system operator, for example, or by automated machine vision technologies. Additional criteria for selecting an appropriate orientation of the oocyte can include, for example, metrics such as total retardance and/or total spindle cross-sectional area calculated by electronic control system **114** for a set of birefringence images.

One method for re-orienting the oocyte is to adjust the air pressure in pipette **250** to allow the oocyte to rotate more freely on the pipette tip, and to nudge the oocyte using a sharp instrument such as an injection pipette to cause the oocyte to rotate. A second method for re-orienting the oocyte is to apply a torque manually to pipette holder **246** in order to effect rotation of the oocyte about axis **290** of sample manipulator **106.** A third method for re-orienting the oocyte includes actuating motor **200,** which applies a torque to inner tube **210** to cause rotation of pipette **250** about axis **290** in a controlled manner. Using the third method, rotation-dependent translations of the oocyte within object plane **150,** and rotation-dependent translations of the equatorial plane of the oocyte away from object plane **150,** can be corrected using a calibration of sample manipulator **106** determined in step **404a,** for example. A birefringence image of the oocyte showing the meiotic spindle is recorded.

Once the oocyte is oriented so that the polar axis of the meiotic spindle lies in object plane **150,** one or more birefringence images of the oocyte are obtained in step **408.** The birefringence image or images can be used to calculate information about the oocyte, such as the magnitude and orientation of optical retardance in regions of the oocyte that correspond to each of the pixels in the birefringence image(s). This information can be used to generate, via processor **120,** a birefringence image display of the oocyte where the intensity of each of the pixels in the birefringence image display corresponds to a magnitude of total optical retardance in spatial regions of the oocyte to which the pixels correspond.

A series of one or more metrics can be calculated, in step **410,** on the basis of birefringence image or images obtained in step **408,** to quantify various properties of the oocyte. The metrics can be used, in step **412,** to assess (either by a system operator or in automated fashion) the developmental potential of the oocyte. Steps **410** and **412** will be discussed in greater detail later.

For certain oocyte samples, it may not be possible to position the polar axis of the oocyte's spindle in object plane **150.** For example, the polar axis of the oocyte's spindle may, by coincidence, be aligned collinearly with axis **290,** so that rotation about axis **290** does not re-orient the spindle's polar axis. In this situation, the oocyte spindle orientation with respect to object plane **150** remains fixed at an angle that is equal to the mounting angle θ.

This systematic approach to aligning a sample with birefringent properties can also be applied to other non-oocyte biological samples, as well non-biological samples.

### V. Assessing Oocyte Developmental Potential

Assessment of an oocyte's developmental potential can be made based on various quantitative metrics calculated from birefringence images of the oocyte. In particular, images of structural elements of the oocyte such as the spindle, zona pellucida, cytoplasm, and oolemma can be used to calculate quantitative metrics that describe particular features and/or properties of these structural elements. The metrics can then be used individually or in combination to assess the developmental potential of the oocyte. Assessment of developmental potential for a particular oocyte can be made by a system operator in view of one or more metrics. Alternatively, or in addition, the assessment can be made in an automated manner by electronic control system **114** according to mathematical algorithms and/or calibration data. Generally, the assessment will be more reliable and accurate when systematic techniques for positioning and orienting the sample within the birefringent imaging system are used, such as those described in the above section.

In some embodiments, assessment of developmental potential can include assigning each oocyte into one of multiple tiers (for example, three or more tiers, such as, *e.g.*, 5 or 10 tiers), the multiple tiers reflecting differing measures of developmental potential. In some embodiments, assessment of developmental potential can include comparing one or more metrics to reference information (such as reference values, like predetermined threshold or limiting values, and/or reference images), and accepting or rejecting an oocyte based on these comparisons. For example, the reference information can be a series of reference images that are birefringent images indicative of oocytes with different developmental potentials (e.g., reference birefringence images corresponding to the different tiers used in an oocyte assessment.) A user and/or the electronic processor by way of automated machine vision techniques can compare the birefringence image of a present sample to those in the reference series to determine the best match and thereby assign a grade corresponding to the that of the matching reference image. Furthermore, in other embodiments, reference values, including threshold values, can be displayed on a reference image using black-white, false-color, or other methods used to visually display information. Moreover, measurement and assessment of subsequent oocyte samples can be made with reference to the same reference information.

Metrics are derived, in general, from quantitative birefringence image data recorded using system **100.** With respect to mammalian oocytes, image data from a number of different structural entities can be used to calculate various assessment metrics.

### A. The Meiotic Spindle

Oocytes that lack a birefringent spindle or that have aberrant spindle morphologies have been shown to have reduced developmental potential. Healthy spindle functioning ensures that primary and secondary oocytes each receive a normal complement of chromosomes. Disruption of the meiotic spindle typically results in abnormal chromosomal alignment such that the resulting daughter cells may have either extra copies of a chromosome or lack the chromosome altogether. Fertilization of oocytes with abnormal spindles can produce aneuploid embryos (embryos that do not have the appropriate number of chromosomes found in healthy individuals of the same species) that may fail to develop. If development does proceed, aneuploid embryos can result in offspring having serious genetic disorders. Spindle integrity can be affected by a variety of factors including maternal age, fluctuations in temperature, length of time the oocyte spends in *in vitro* culture, osmotic stress, and chemical agents that disrupt microtubules.

A number of features of spindle morphology can provide useful information about the functioning of the meiotic spindle, and by inference, the developmental potential of the oocyte. Metrics relating to spindle features can generally be grouped into three categories: (1) metrics relating to spindle size and shape; (2) metrics relating to spindle location; and (3) metrics relating to spindle retardance.

A normal healthy meiotic spindle is symmetrically oval in shape, although absolute sizes of spindles can differ among organisms. One measure of spindle size is length of the meiotic spindle. Any measurement that is related to the polar separation, *e.g.*, a separation between the outer ends of the spindle along the polar axis, can be used to calculate spindle length. For example, in a birefringence image of an oocyte showing a meiotic spindle, locations of the spindle poles can be determined using automated image analysis and/or machine vision software. A length of the spindle can be measured along an axis connecting the poles.

Another metric derived from the shape of the spindle dimension is the spindle width. The spindle width can be determined by measuring a distance along a line perpendicular to the polar axis from a first point on one side of the spindle perimeter (in cross-section) to a point on the opposite side of the spindle perimeter. The spindle width can be measured at any point along the polar axis, *e.g.*, through a mid-point of the polar axis, or at any point between the mid-point and (including) the poles.

The total cross-sectional area of the meiotic spindle can also be used as an assessment metric for oocyte developmental potential. Total spindle cross-sectional area can be calculated either by direct measurement (*e.g.*, pixel counting) or by fitting the spindle region with a geometric shape, *e.g.*, an ellipse or a rectangle, that encompasses the spindle and calculating an area of the fitted shape. A total length of the spindle perimeter can also be determined from a geometric shape fitted to the spindle region, and provides another assessment metric. Calculation of any of the above spindle-based metrics can also include a conversion from image pixels to distance units based on a distance calibration.

Metrics relating to spindle shape can also provide indicators of oocyte developmental potential. Oocytes that include asymmetric or misshapen spindles that deviate from the normal "barrel-shaped" morphology are at higher risk for generating aneuploid progeny. A spindle symmetry-based metric can be calculated, for example, by determining whether the spindle poles lie along a symmetry line of a geometric shape such as an ellipse fitted to the spindle, and calculating a deviation angle between a line connecting the poles and the symmetry line of the fitted geometric shape. Symmetry-based metrics can also be calculated using measurements such as the distance between the symmetry line and each of the poles, for example, or by comparing spindle widths at each of the poles. Metrics based on spindle eccentricity can be determined by calculating a ratio of the lengths of the major and minor axes of an ellipse fitted to the spindle region in a birefringent image.

The location of a spindle within an oocyte can also be informative of oocyte developmental potential. Meiotic cell divisions of the oocyte are typically highly asymmetric, resulting in formation of a large oocyte and a smaller polar body. This asymmetry is mediated by proper positioning of the meiotic spindle close to the oolemma, in a region called the cortex. Oocytes with improperly oriented spindles are at risk for generating aneuploid progeny. Thus, metrics derived from measurements of spindle position and orientation can be used to assess the developmental potential of oocytes.

The location of the spindle relative to the oolemma can be determined by measuring a position of the spindle along a radial line from the center of the oocyte to the oolemma. The orientation of the polar axis of the spindle relative to the oocyte is also an measure of spindle functioning. The polar axis is typically radially oriented in a healthy human oocyte. Thus, the orientation of the spindle's polar axis provides an additional assessment metric. The polar axis of the spindle can be determined as above, and its position relative to a radius of the oocyte can be calculated, for example, by calculating an angle between the polar axis and an oocyte radial line.

The location of the spindle relative to the polar body can also be indicative of oocyte developmental potential, and can therefore provide an assessment metric. For example, a deviation angle between a line connecting a center of the oocyte with the spindle and a line connecting the center of the oocyte with a center of the polar body can be calculated and used to derive a quantitative assessment metric.

Spindle retardance can provide additional useful metrics for evaluation of oocyte developmental potential. Spindle retardance provides a quantitative index of spindle functioning. The microtubules that comprise the spindle are highly ordered and thus birefringent; conditions that change microtubule organization can therefore change the birefringence of the spindle and can be indicative of oocytes that are at risk for generating aneuploid progeny. One measure of spindle retardance can include determining the integrated retardance of the spindle by summing all the retardance values for pixels contained within a perimeter determined, for example, by an edge-detection algorithm. Another metric derived from measurements of retardance includes determining the uniformity of retardance orientation across the cross-sectional area of the spindle.

Spindle retardance measurements can also provide metrics relating to spatial variation in retardance values across the cross-sectional area of the spindle. Spatial variations in retardance can be evaluated, for example, by determining a distribution of spatial frequencies of retardance values along the polar axis of the spindle, along a direction perpendicular to the polar axis, or by a measure that computes a two-dimensional distribution of spatial frequencies of retardance values such as a two-dimensional spatial Fourier transform.

### B. The Zona Pellucida

Oocytes with zona defects have been shown to have reduced developmental potential. The capacity of such oocytes to be fertilized may be compromised. In addition, zona morphology, particularly the degree of irregularity and the thickness, appear to be important for the development of a zygote. For example, if the zona is too thin, the embryo can disaggregate; if the zona is too thick, the zygote may fail to hatch.

A number of features of zona morphology can provide useful information about the functioning of the zona, and by inference, metrics for assessing the developmental potential of the oocyte. These metrics relate generally to zona thickness, zona retardance, and zona uniformity.

Several different types of measurements can be taken as indices of zona thickness, and can therefore provide assessment metrics. One such measurement is an absolute thickness measurement of the entire zona, of the inner birefringent layer, or of the outer birefringent layer. Alternatively, relative thicknesses of any or all of the layers can also be calculated to provide metrics. In many embodiments, measurements of zona thickness can be determined in automated fashion using image analysis algorithms such as edge-detection algorithms operating directly on birefringence images of the oocyte. Thickness measurements can further include a conversion from measurements in units of image pixels to measurements in distance units based on a calibration of system **100.**

Zona retardance can also provide useful metrics for evaluation of oocyte developmental potential. As for spindle retardance, measurement of zona retardance provides a quantitative index of zona functioning. Molecular changes within the zona that alter the birefringence properties of the zona help to identify oocytes that are at risk for producing developmentally compromised progeny as well as to identify embryos that are unlikely to survive until implantation. Metrics relating to zona retardance can be determined in a variety of ways. For example, metrics can include calculation of zona retardance as a maximum retardance among all pixels within the zona's cross sectional area. Other metrics can be derived from an average peak retardance of the zona, which can be determined by averaging peak retardance values in radial directions of the zona along two or more different radial lines. Similarly, maximum retardance and average peak retardance of each of the individual birefringent layers can also be calculated, and can provide assessment metrics for oocytes.

A degree of irregularity and/or asymmetry in zona shape may also correlate with oocyte developmental potential. For example, an angular variation in zona shape (*e.g.*, curvature or thickness) can provide assessment metrics which relate specifically to zona asymmetry. As above, metrics applicable to the zona as a whole can also be applied to any of the identified layers within the zona.

### C. The Cytoplasm

The cytoplasm of an oocyte can also be used to assess oocyte developmental potential. Spatial variation in the birefringence properties of the cytoplasm (or the absence thereof) can be indicative of the general health of an oocyte. Metrics derived from measurements of oocyte cytoplasm can include measurements of birefringence characteristics of the cytoplasm such as average retardance, determined by averaging retardance values for all pixels within a region corresponding to the cytoplasm in a birefringence image. Metrics can also relate to a distribution of spatial frequencies in a retardance variation pattern across the cross-sectional area of the cytoplasm, calculated using mathematical techniques such as a two-dimensional spatial Fourier transform, for example, or coincidence matrices, or other metrics which assess sample texture in an image.

### D. The Oolemma

The oolemma is a structure that has measurable retardance, which is evident just inside the zona pellucida in a cross sectional view of the oocyte. The magnitude, uniformity, and dimensions of the retardance can be used as metrics for assessing oocyte health.

### E. The Polar Body

The polar body's location relative to the polarity of the oocyte, as determined by the orientation of the spindle inside the oocyte, can also reveal information about the state of the oocyte. The relative position of the polar body to the oocyte polarity can be determined by manipulating the oocyte to bring the spindle into focus first and then the polar body second, while noting the change in angle of rotation about the holding pipette axis between images where these structures are in focus. In some cases, both the polar body and the spindle will be in focus in the same image.

Metrics disclosed herein can be useful in assessing oocyte developmental potential in therapeutic applications of assisted reproductive technologies, which include methods used to achieve pregnancy by artificial or partially artificial means. Infertility, which refers to an inability to naturally conceive a child and/or an inability to carry a pregnancy to term, can result from oocyte-related or sperm-related factors. A number of therapeutic approaches in infertility involve recovery of and *in vitro* manipulation of oocytes. The metrics disclosed herein can be used to evaluate oocyte quality prior to *in vitro* fertilization and intracytoplasmic sperm injection. In addition, various metrics can be used to monitor embryo quality prior to transfer of the embryo back to its maternal host.

In addition to the various metrics used individually, combinations of metrics can be employed to assess developmental potential of oocytes. For example, various metrics can be combined according to mathematical algorithms that weigh the metrics differently to produce a composite metric for evaluating oocytes. In some embodiments, various metrics can be checked against predetermined ranges for each of the metrics, and an oocyte can be rejected if even one of the metrics falls outside its predetermined range. In certain embodiments, oocytes can be graded into tiers based upon a scheme that includes multiple different metrics, each of which influences the final grading assignment for a particular oocyte.

### VII. Assisted Reproductive Technology (ART) and Other Applications

The apparatus, systems, and methods disclosed herein can generally be used to provide accurate, non-invasive, and rapid assessment of oocyte developmental potential. For example, the apparatus, systems, and methods can be used in assisted reproductive technologies, in reproductive toxicology analysis, and in the development of stem cell lines.

A commonly used method in ART is *in vitro* fertilization (IVF), in which eggs are fertilized outside the body in a laboratory dish. IVF is a multi-step process that includes a number of variants and adjunct therapies, each of which entails relatively extensive manipulation of unfertilized and/or fertilized oocytes. Since the success of IVF depends, in part, upon the selection of oocytes with optimum developmental potential as well as the use of laboratory methods for handling oocytes that ensure maintenance of developmental potential, systematic monitoring of oocyte developmental potential during the IVF process can contribute to a favorable IVF outcome.

IVF was first performed on humans in 1978 and although the practice has become more routine, it remains a relatively invasive, complex and expensive procedure that does not always result in pregnancy. Percentages of IVF cycles that result in live births can range, depending upon the age of the mother, from about 43% for women under 35 years of age to about 18% for women aged 41-42, for example. One complication of IVF is the relatively high rate of multiple births. To increase the odds of pregnancy, multiple eggs can be fertilized and often multiple embryos are transferred back to the mother. Multiple births can be associated with increased pregnancy loss, premature labor, obstetrical complications, prematurity, and neonatal morbidity with the potential for long term damage. Therefore, methods that provide for careful, rapid assessment of oocyte developmental potential may contribute to an increased success rate for IVF while at the same time, may potentially help to alleviate the relatively high rate of multiple births associated with IVF.

Indications for IVF can stem from both female and male-related factors. These factors include: defects in the woman's anatomy that prevent normal fertilization, *e.g.*, blocked, damaged or absent Fallopian tubes; prolonged egg-related infertility that has not responded to other methods of treatment; premature menopause so that pregnancy is only possible using donor eggs; male-related factors such as extremely low sperm counts or abnormal sperm morphology; and instances when one or both parents are known carriers of certain genetic diseases, *e.g.*, Duchennes muscular dystrophy, Tay-Sachs disease, cystic fibrosis, and myotonic dystrophy, so that preimplantation genetic analysis of the zygote may be of value. IVF also has veterinary applications and has been used to breed economically valuable domestic animals, *e.g.*, cows and horses, as well as to propagate endangered species, *e.g.,* European mouflon, bongo antelope, Javan banteng (*Bos javanicus),* the marbled cat *(Pardofelis marmorata)* and the serow *(Capricornis sumatraensis).*

IVF is typically carried out by treating the female, *e.g.*, a human female or female of any non-human species, with hormones to insure the production of oocytes, surgically collecting the oocytes, culturing the oocytes and sperm in laboratory media, and transferring the fertilized oocytes back into the uterus of a suitable recipient. In humans, the recipient can be the same individual from whom the oocytes were collected or, in cases where donor eggs are used, the recipient can be another individual who is genetically unrelated to the donor. Animal embryos are often transferred into surrogate recipients, *e.g.*, a different individual than the one from which the oocytes were collected and embryos from endangered species are sometimes transferred to recipients of another biological species altogether, such as a closely related domestic animal. Oocytes can be collected as secondary, metaphase II arrested oocytes or as primary oocytes that are then induced to complete meiosis *in vitro.* A period of about 3-5 days can elapse between the time of oocyte retrieval and transfer into a recipient. During this period of *in vitro* culture, the oocyte is particularly vulnerable to detrimental environmental effects, *e.g.*, fluctuations in temperature and fluctuations in the pH of the culture medium, that can compromise developmental potential.

The apparatus, systems, and methods provided herein for assessing oocyte developmental potential can be used at many points in the IVF procedure. The non-invasive aspect of the methods ensures that the same oocyte can be serially evaluated over time, so that the developmental progress of individual oocytes can be followed systematically. For example, oocyte developmental potential can be analyzed immediately following retrieval of the oocytes, upon fertilization and for evaluation of fertilized oocytes prior to transfer into a recipient (*e.g.*, to the 8-cell stage or up to blastocyst), and at other points in time during oocyte *in vitro* culture for assessing oocyte health.

Male infertility characterized by extremely low sperm counts, abnormal sperm motility, or abnormal sperm morphology, can be treated by a variant of IVF known as intracytoplasmic sperm injection (ICSI). In ICSI, oocytes are collected as in standard IVF, but instead of incubating the oocytes in sperm containing media, a single sperm is injected directly into an oocyte. A holding pipette is used to stabilize the mature oocyte and from the opposite side, a thin, hollow needle containing a sperm is pierced through the oolemma into an inner portion of the oocyte. Orientation of the microinjection needle in ICSI is important in order to avoid damage to the meiotic spindle that may result in abnormal chromosome separation. The metrics disclosed herein can be used for oocyte evaluation for ICSI. For example, assessments based on one or more metrics calculated from birefringence images can be used to select oocytes for ICSI that appear to have the best developmental potential, and can further be used for careful assessment of development of the fertilized oocyte following ICSI. In addition, the apparatus and systems can be used to accurately position oocytes for sperm injection.

IVF is also used in conjunction with preimplantation genetic diagnosis (PGD). PGD can be used when one or both parents carries a genetic mutation that can potentially be transmitted to their offspring, and/or in cases of recurrent pregnancy loss due to aneuploidy. PGD can be carried out on unfertilized and fertilized oocytes. Oocytes can be screened for the presence or absence of a particular mutation by removing either the first or second polar body and probing the polar body for specific genetic defects, *e.g.*, cystic fibrosis, B thalassemia, Tay-Sachs disease, sickle cell disease, and Huntington's disease, with polymerase chain reaction (PCR) technology. Alternatively, specific chromosomal defects, *e.g.*, translocations or aneuploidies can be probed using fluorescent in situ hybridization (FISH). In addition, FISH can determine the presence of most common aneuploidies that are the cause of implantation failure or early pregnancy loss. PCR and/or FISH technologies can also be employed after fertilization. Typically, a biopsy is performed after a few cell divisions have taken place. A single cell (a "blastomere") can be removed from an 8-cell embryo for testing without compromising the biopsied embryo. When performed after fertilization, FISH and PCR technology can be used to identify the gender of the embryo in order to screen for embryos carrying sex-linked genetic abnormalities. For example, FISH can used to deselect male embryos in situations where the mother is a carrier of an X-linked disorder or harbors fragile X syndrome.

PCR or FISH analysis may take up to two days, and depending upon the results, an analyzed oocyte or embryo may or may not be selected for fertilization or transfer, respectively. The apparatus, systems, and methods disclosed herein can be used in conjunction with PGD methods, both for assisting in the selection of individual oocytes and/or embryos for PGD, as well as for monitoring the health and developmental potential of oocytes and embryos following polar body or blastomere biopsy.

In most IVF procedures, more oocytes are collected and more embryos are generated that can be safely implanted back into the mother in one cycle. These "supernumerary" oocytes and/or embryos are typically frozen for fertilization and/or implantation at a later date, if desired. Embryos and oocytes can be preserved by a specialized freezing process known as cryopreservation, a process where cells or whole tissues are preserved by cooling to temperatures below 0 °C, such as (typically) -80 °C or -196 °C. At these temperatures, biological activity, including the biochemical reactions that would lead to cell death, is effectively stopped. Frozen tissues are vulnerable to damage during the freezing and thawing processes, so eggs and embryos are usually frozen in the presence of a cryoprotectant solution. Cryopreservation of oocytes, on the other hand, remain at a fairly low success rate. New protocols are currently being explored. The apparatus, systems, and methods disclosed herein can be used to assess the developmental potential of frozen eggs and embryos.

Other applications of the apparatus, systems, and methods include reproductive toxicology analysis and the development of embryonic stem cell lines. "Reproductive toxicology", as used herein, refers to the study of, and methods for, analysis of substances with potentially harmful effects with respect to gametes and developing embryos. Classes of chemical compounds that can have deleterious effects on developing organisms may alter metrics derived from oocyte images in a systematic, quantifiable fashion. Thus, the apparatus, systems, and methods disclosed herein can be used to evaluate effects of particular chemical compounds on oocyte developmental potential. For example, analysis of the effects of specific compounds on the developmental potential of oocytes from laboratory animals can provide information about the relative potential of such compounds to induce aneuploidy in humans.

Embryonic stem cell lines are cultured cells obtained from preimplantation embryos. Embryonic stem cells are "totipotent", that is, they retain the capacity to differentiate into an extremely wide range of functional adult cell types. Embryonic stem cells represent a potential source of replacement cells to treat numerous diseases. Any disease in which there is tissue degeneration can be a potential candidate for stem cell therapies, including conditions and disabilities such as Parkinson's and Alzheimer's diseases, spinal cord injury, stroke, bums, heart disease, Type 1 diabetes, osteoarthritis, rheumatoid arthritis, muscular dystrophies, and liver diseases.

Embryonic stem cell lines have been produced by harvesting a few cells from the inner cell mass of the blastocyst. One factor limiting the potential of such embryonic stem line lines is the immune response mounted by the recipient against foreign tissue. Embryonic stem cell lines derived from an individual's own tissue may provide starting material for replacement tissue that does not provoke a deleterious immune response. Such embryonic stem cell lines can be generated by somatic cell nuclear transfer (SCNT). In SCNT, genetic material is removed from a donor oocyte by aspirating either the germinal vesicle or the meiotic spindle, and the nucleus from a somatic cell of a patient is microinjected into the enucleated oocyte. The apparatus, systems, and methods disclosed herein can be used to monitor the health of recipient oocytes, and to follow the developmental progress of microinjected oocytes.

### VIII. Other Imaging Modalities

The methods, apparatus, and systems disclosed herein can be used with imaging modalities other than birefringence imaging. For example, fluorescence imaging can be used to,derive information about samples and, in some cases, to assess developmental potential of such samples. Samples having fluorescent structural entities can be oriented using sample manipulator **106,** for example, in order to enhance contrast and/or resolution in fluorescence images of the samples.

### IX. Optical Systems

The optical apparatus and systems disclosed herein can include, in general, a variety of optical elements and devices for manipulating and altering light produced by optical source **102.** Optical source **102** can be a lamp for example, such as an incandescent lamp, or a halogen lamp, or a fluorescent lamp. Alternatively, optical source **102** can include one or more light-emitting diodes. In some embodiments, optical source **102** can include a laser source such as a continuous-wave laser source or a pulsed laser source. In certain embodiments, optical source **102** can include multiple different elements, such as multiple different diodes, operating in concert to produce source light **130** having a distribution of light wavelengths. Optical source **102** can further include optical elements such as lenses, waveplates, polarizing elements, and other elements for tailoring the properties of source light **130.** Optical source **102** can include electronic devices such as power supplies and control electronics for operating various light emitting elements. Some or all of these electronic devices can transmit signals to and receive signals from electronic control system **114** via communication line **122a.**

Illumination optics **104** and detection optics **110** can include similar types of optical elements. For example, illumination optics **104** and detection optics **110** can include light focusing and redirecting light such as lenses and mirrors. Illumination optics **104** and detection optics **110** can also include filtering elements such as, for example, bandpass filters, interference filters, notch filters, and other types of filters for manipulating the spectral properties of light. Further, illumination optics **104** and detection optics **110** can include optical elements for manipulating the polarization properties of light, such as polarizers, waveplates, and liquid crystal devices. Some of these elements may be automatically configurable via electronic signals sent from electronic control system **114** along communication lines **122b** and **122c.**

Detector system **112** (also referred to herein as a "camera") can include one or more CCD or CMOS detector arrays, for example, configured to measure an intensity of detection light **136** transmitted through detection optics **110.** Detector system **112** can receive control signals from electronic control system **114** via communication line **126,** to control the timing and duration of exposure, or other aspects of its operation; and it can also transmit image data to processor **120** through communication line **126.**

### X. Electronic Processing and Software

The steps described above in connection with various methods for collecting, processing, analyzing, interpreting, and displaying information from samples (such as birefringence images of biological samples) can be performed by electronic processors (such as computers or preprogrammed integrated circuits) executing programs based on standard programming techniques. Such programs are designed to execute on programmable computers or specifically designed integrated circuits, each comprising a processor, a data storage system (including memory and/or storage elements), at least one input device, and at least one output device, such as a display or printer. The program code is applied to input data (*e.g.*, images from a detector) to perform the functions described herein and generate output information (*e.g.*, birefringence images of samples, assessment metrics relating to structural elements of samples, etc.), which is applied to one or more output devices. Each such computer program can be implemented in a high-level procedural or object-oriented programming language, or an assembly or machine language. Furthermore, the language can be a compiled or interpreted language. Each such computer program can be stored on a computer readable storage medium (*e.g.*, CD ROM or magnetic diskette) that, when read by a computer, can cause the processor in the computer to perform the analysis and control functions described herein.

### OTHER EMBODIMENTS

A number of embodiments of the invention have been described. Nevertheless, it will be understood that various modifications may be made without departing from the spirit and scope of the invention. Accordingly, other embodiments are within the scope of the following claims.

## Claims

1. A method comprising:
assessing the developmental potential of an oocyte based on one or more quantitative metrics derived from one or more birefringence images of a sample comprising the oocyte, wherein the assessing comprises comparing the one or more quantitative metrics to reference information.

2. The method of claim 1, wherein the reference information is derived from one or more birefringence images of a reference sample, wherein the birefringence images of the first mentioned sample and the reference sample are obtained under like conditions.

3. The method of claim 2, wherein the reference information comprises one or more reference values.

4. The method of claim 2, wherein the reference information comprises one or more reference images.

5. The method of claim 1, further comprising assessing the developmental potential of an additional oocyte sample based on one or more quantitative metrics derived from one or more birefringence images of the additional oocyte, wherein the assessing comprises comparing the one or more quantitative metrics for the additional oocyte to the same reference information as that for the assessment of the first mentioned oocyte sample.

6. The method of claim 1, wherein the one or more quantitative metrics are derived from a birefringence image of at least one structural entity in the oocyte.

7. The method of claim 6, wherein the structural entity is an organelle of the oocyte.

8. The method of claim 7, wherein the organelle is a meiotic spindle.

9. The method of claim 7, wherein the organelle is a zona pellucida.

10. The method of claim 6, wherein the structural entity is at least one of the structures selected from the group consisting of a meiotic spindle, a zona pellucida, a cytoplasm, an oolemma, and a polar body.

11. The method of claim 1, wherein assessing the developmental potential of the oocyte comprises grading the oocyte into one of three or more tiers based on the one or more quantitative metrics.

12. The method of claim 3, wherein the one or more reference values comprise one or more predetermined thresholds.

13. The method of claim 12, wherein the one or more predetermined thresholds are provided by a user prior to obtaining the birefringence image.

14. The method of claim 1, wherein the assessment is made automatically by an electronic processor.

15. The method of claim 1, wherein the assessment is made based on a combination of quantitative metrics.

16. The method of claim 15, wherein the quantitative metrics are combined according to a mathematical algorithm.

17. The method of claim 1, wherein the one or more quantitative metrics comprises any of a spatial location and a size of a structural entity of the oocyte in the birefringence image.

18. The method of claim 1, wherein the one or more quantitative metrics comprises dimensional information about a meiotic spindle of the oocyte.

19. The method of claim 1, wherein the one or more quantitative metrics comprises dimensional information about a zona pellucida of the oocyte.

20. The method of claim 1, wherein the one or more quantitative metrics comprises dimensional information about a cytoplasm of the oocyte.

21. The method of claim 1, wherein the one or more quantitative metrics are determined automatically by an electronic processor.

22. The method of claim 1, wherein the one or more birefringence images are obtained using a birefringence imaging system comprising a microscope.

23. The method of claim 1, wherein the one or more birefringence images are obtained with a polar axis of a meiotic spindle of the oocyte substantially aligned in an object plane of a birefringence imaging system used to obtain the one or more birefringence images.

24. The method of claim 1, wherein the one or more birefringence images are obtained with a polar axis of a meiotic spindle of the oocyte forming an angle with respect to an object plane of a birefringence imaging system used to obtain the birefringence images, and wherein the angle is less than about 30 degrees.

25. The method of claim 1, wherein the one or more birefringence images are obtained with an equatorial plane of the oocyte substantially aligned with an object plane of a birefringence imaging system.

26. The method of claim 25, wherein a cross-sectional area of the oocyte is a local maximum in a birefringence image obtained when the equatorial plane of the oocyte and the object plane of the birefringence imaging system are substantially aligned.

27. The method of claim 26, wherein the oocyte is substantially spherical.

28. An apparatus comprising:
a camera for use with a microscope birefringence imaging system to obtain a birefringence image of a sample comprising an oocyte; and
an electronic processor coupled to the camera and configured to store reference information for use in assessing the developmental potential of the oocyte based on one or more quantitative metrics derived from the birefringence image, wherein the assessing comprises comparing the one or more quantitative metrics to the reference information.

29. The apparatus of claim 28, wherein the electronic processor is configured to calculate the one or more quantitative metrics.

30. The apparatus of claim 28, wherein the electronic processor is configured to perform the comparison of the one or more quantitative metrics to the reference information to make the assessment.

31. The apparatus of claim 28, wherein the reference information is derived from one or more birefringence images of a reference sample, wherein the birefringence images of the first mentioned sample and the reference sample are obtained under like conditions.

32. The apparatus of claim 31, wherein the reference information comprises one or more reference values.

33. The apparatus of claim 31, the reference information comprises one or more reference images.

34. The apparatus of claim 28, wherein the quantitative metrics are derived from a birefringence image of at least one structural entity in the oocyte.

35. The apparatus of claim 30, wherein the electronic processor is configured to grade the oocyte into one of three or more tiers based on the one or more quantitative metrics.

36. The apparatus of claim 32, wherein the one or more reference values comprises one or more predetermined thresholds.

37. The apparatus of claim 28, wherein the one or more quantitative metrics comprise dimensional information about a structural entity of the oocyte.

38. The apparatus of claim 37, wherein the dimensional information comprises at least one of a size, a length, an area, an orientation, a position, and a presence or absence of the structural entity.

39. The apparatus of claim 28, wherein the one or more quantitative metrics comprise retardance information about a structural entity of the oocyte.

40. The apparatus of claim 28, wherein the birefringence image is obtained with a polar axis of a meiotic spindle of the oocyte substantially aligned in an object plane of the imaging system.

41. The apparatus of claim 28, wherein the birefringence image is obtained with a polar axis of a meiotic spindle of the oocyte forming an angle with respect to an object plane of the imaging system, and wherein the angle is less than about 30 degrees.

42. The apparatus of claim 28, wherein the birefringence image is obtained with an equatorial plane of the oocyte substantially aligned with an object plane of the imaging system.

43. The apparatus of claim 28, further comprising the microscope birefringence imaging system.
